# NEW EUROPEAN PATENT SPECIFICATION

(11) **EP 0 567 566 B2**
(45) Date of publication and mention of the opposition decision: **04.07.2007**
(45) Mention of the grant of the patent: 15.03.2000
(21) Application number: 92904429.5
(22) Date of filing: 17.01.1992
(51) Int. Cl.: C07K 14/715, A61K 38/02

(54) **METHODS FOR TREATING TUMOR NECROSIS FACTOR MEDIATED DISEASES**
METHODEN ZUR BEHANDLUNG VON DURCH DEN TUMOR NEKROSE FAKTOR AUSGELÍSTEN KRANKHEITEN
PROCEDES POUR TRAITER LES MALADIES INDUITES PAR FACTEUR DE NECROSE TUMORALE

(30) Priority: 18.01.1991 US 644345
(43) Date of publication of application: 03.11.1993
(73) Proprietor: Amgen Inc.,, Thousand Oaks, California 91320-1799 (US)
(72) Inventor: CARMICHAEL, David, F., Boulder, CO 80302-9345 (US); SMITH, Christopher, G., Eldorado Springs, CO 80025 (US); THOMPSON, Robert, C., Boulder, CO 80303 (US); RUSSELL, Deborah, Boulder, CO 80303 (US); KOHNO, Tadahiko, Louisville, CO 80027 (US)
(74) Representative: Vogelsang-Wenke, Heike
(86) International application number: PCT/US1992/000432
(87) International publication number: WO 1992/013095

(56) References cited:
- EP-A- 0 247 860
- EP-A- 0 308 378
- EP-A- 0 393 438
- EP-A- 0 422 339
- WO-A-90/13575
- WO-A-92/16221
- DE-A- 3 910 323
- PROCEEDINGS OF THE NATIONAL ACADEMY OF SCIENCES,USA vol. 87 , November 1990 , WASHINGTON D.C.,USA pages 8331 - 8335 KOHNO ET AL 'A SECOND TUMOR NECROSIS FACTOR RECEPTOR GENE PRODUCT CAN SHED A NATURALLY OCCURRING TUMOR NECROSIS FACTOR INHIBITOR'
- THE JOURNAL OF BIOLOGICAL CHEMISTRY vol. 265, no. 3 , 25 January 1990 , BALTIMORE,USA pages 1531 - 1536 ENGELMANN ET AL 'TWO TUMOR NECROSIS FACTOR-BINDING PROTEINS PURIFIED FROM HUMAN URINE'
- CHEMICAL ABSTRACTS, Volume 113, issued 1990, DAYER et al., "Purification and Characterization of Human Tumor Necrosis Factor alpha Inhibitor", page 454, see Abstract No. 38760n, DE, A, 3,910,323 (1989).

## Description

### BACKGROUND OF THE INVENTION BACKGROUND OF THE INVENTION

The present invention relates to a pharmaceutical composition comprising a TNF inhibitor modified by the addition of a high molecular weight repeating polymeric moiety material as well as to the use of a therapeutically effective amount of a TNF inhibitor for the preparation of a medicament for treating or preventing osteoarthritis, ankylosing spondylosis, gonococcal arthritis. Reiter's disease arthritis and gout.

Tumor Necrosis Factors (TNFs) are a class of cytokines produced by numerous cell-types, including monocytes and macrophages. At least two TNFs have been previously described, specifically TNF alpha and TNF beta (lymphotoxin).

These known TNFs have important physiological effects on a number of different target cells involved in the inflammatory response. The proteins cause both fibroblasts and synovial cells to secrete latent collagenase and prostaglandin E2, and cause osteocyte cells to stimulate bone resorption. These proteins increase the surface adhesive properties of endothelial cells for neutrophils. They also cause endothelial cells to secrete coagulant activity and reduce their ability to lyse clots. In addition they redirect the activity of adipocytes away from the storage of lipids by inhibiting expression of the enzyme lipoprotein lipase.

TNFs cause hepatocytes to synthesize a class of proteins known as "acute phase reactants" and they act on the hypothalamus as pyrogens. Through these activities, it has been seen that TNFs play an important part in an organism's response to a variety of indications such as infection and injury. See. e.g., articles by P.J. Selby et al., Lancet, February 27, 1988, pg. 483; H.F Starnes, Jr. et al., J. Clin Invest., vol. 82, pg. 1321 (1988): A. Oliff et al. Cell, vol. 50, pg. 555 (1987); and A. Waage et al., Lancet, February 14, 1987, pg. 355.

A disease is considered to be a "TNF mediated disease" if the spontaneous or experimental disease is associated with elevated levels of TNF in bodily fluids or in tissues adjacent the focus of the disease or indication within the body. In many cases, such TNF mediated diseases are also recognized by the following additional two conditions: (1) pathologic findings associated with the disease can be mimicked experimentally in animals by the administration of TNF; and (2) the pathology induced in experimental animal models of the disease can be inhibited or abolished by treatment with agents which inhibit the action of TNF. In most "TNF mediated diseases" at least two of the three conditions are met, and in many "TNF mediated diseases" all three conditions are met.

A list of diseases which satisfy these criteria includes, but is not limited to, the following;
1) adult respiratory distress syndrome
2) pulmonary fibrosis
3) arthritis
4) inflammatory bowel disease
5) septic shock

Evidence for the TNF-mediation of these five diseases will be presented. Animal models of septic shock, arthritis and adult respiratory distress syndrome are well known, and are used herein to demonstrate the ability of TNF inhibitors to treat TNF-mediated disease.

Adult respiratory distress syndrome (ARDS) is characterized by the rapid onset of dyspnea, tachypnea, cyanosis, severe hypoxemia, decreased lung compliance, and increased pulmonary vascular permeability. Mortality in ARDS is in excess of 50%. Risk factors associated with the development of ARDS include trauma, massive blood transfusion, disseminated intravascular coagulation, oxygen toxicity, inhalation of toxins and irritants, and systemic reaction to sepsis, hemorrhagic pancreatitis, burns, and complications of abdominal surgery. A variety of mediators such as prostaglandins, leukotrienes, complement, and free oxygen radicals have been implicated in the causation of the syndrome; however, various treatments designed to inhibit the actions of specific mediators have not improved the clinical course of ARDs. Results of animal experimentation support the hypothesis that TNF is a mediator of ARDS as follows:
1. The infusion of TNF to rats mimics ARDS. After infusion with TNF, pulmonary compliance is reduced, pulmonary water content and cellularity are increased, punctate hemorrhages may be visible grossly and histologic evidence of neutrophil thrombi and diffuse alveolar damage are present. See. E. Ferrari-Baliviera et al., Arch. Surg., vol. 124, pp. 1400-1405 (1989); KJ. Tracey et al., Science, vol. 234, pp. 470-474 (1986).
2. Pretreating rats with anti-TNF antiserum inhibited the induction of an animal model of ARDS. ARDS was induced by hepatic ischemia followed by reperfusion of the liver. Elevated circulating levels of TNF after the reperfusion were presumed to arise from the large mass of fixed hepatic macrophages. Pulmonary capillary leakage was reduced in treated rats compared to values in rats given serum without TNF-blocking properties. See. LM. Colletti et al., Transplantation, vol. 49, pp. 268-272 (1990).
3. In two clinical studies, TNF concentrations were measured in broncho-pulmonary secretions of patients with ARDS. In the first study of five ARDS patients, TNF concentrations exceeded 12.5 ng/mL. No TNF was detected in control samples. In the second study, significant TNF levels were measured in bronchoalveolar lavage fluid of 3 out of 4 patients with ARDS but were below the limit of detection in controls. See, A.B. Millar et al., Lancet. vol. 2, pp. 712-714 (1989); D.J. Roberts et al., Lancet, vol. 2, pp. 1043-1044 (1989).

Pulmonary fibrosis occurs during the end stage of various pulmonary diseases. The fibrosis results from the growth of fibroblasts and an increase of collagen deposition within the alveolar walls. TNF apparently plays a key role in the development of pulmonary fibrosis. TNF is a growth factor of normal diploid fibroblasts at picomolar concentrations. See. B.J. Sugarman et al., Science, vol. 230, pp. 943-945 (1985). Results of animal experimentation involving pulmonary injury induced by bleomycin, a chemotherapeutic agent for cancer, provide evidence supporting that proposal. The TNF-mediation of pulmonary fibrosis is supported by the following:
1. The intravenous infusion of TNF induces diffuse alveolar damage, notably with necrosis of alveolar epithelial and endothelial cells and marked thickening of the alveolar membranes. The subcutaneous infusion of TNF to rats leads to a marked increase of fibroblast proliferation and collagen deposition. See. K.J. Tracey et al., Science vol. 234, pp. 470-474 (1986); P.F. Piguet et al., lnt. Arch. Allerg. Apol. Immunol., vol. 83, p. 18 (1986).
2. The administration of rabbit anti-TNF antibody to mice prevented bleomycin-induced alveolar damage, growth of fibroblasts, and collagen deposition. See. Pierre F Piguet et al., J. Exp Med., vol. 170, pp. 655-663 (1989).
3. A common sequela of patients surviving ARDS is pulmonary fibrosis. During the acute phase of ARDs, TNF is present in significant amounts in the bronchoalveolar fluid.

Arthritis is a term used to describe a variety of indications that are characterized by chronic inflammation in the joints. Included within the definition of the term arthritis are the following: rheumatoid arthritis, osteoarthritis, ankylosing spondylitis, gonococcal arthritis, Reiter's disease arthritis and gout. Evidence is presented below for the mediating role of TNF in rheumatoid arthritis.

Rheumatoid arthritis is a chronic autoimmune disease causing destruction of articular cartilage in the joints. The synovial lining is chronically inflamed and undergoes progressive fibrotic growth. In vitro, TNF activates both the endothelium and leukocytes to promote leukocyte adhesion and stimulates resorption and inhibits synthesis of proteoglycans in cartilage. See, J. R. Gamble et al., Proc. Natl. Acad. Sci USA., vol. 82, pp. 8667-8673 (1985); J. Saklatvala, Nature, Vol. 322, pp. 547-552 (1986). These in vitro activities strongly suggest that TNF may be a mediator of joint pathology in rheumatoid arthritis. The TNF-mediation of rheumatoid arthritis is supported by the following:
1. The intra-articular injection of TNF into the joint of a rabbit causes infiltration of leukocytes into the joint but no proteoglycan loss from cartilage. Injection of submaximal doses of TNF and interleukin-1 (IL-1) into the joint caused a synergistic accumulation of neutrophils. See, B. Henderson et al., Clin, Exp, Immunol., vol. 75, pp. 306-310 (1989). In contrast, in another series of experiments using rabbits, a single intra-articular injection of TNFα caused both cellular infiltration and cartilage proteoglycan loss. Levels of substance P, an inflammation neuropeptide, were increased in the synovial fluid. See. A.S. Rubin et al., Arthritis and Rheumatism, vol. 33, pp. 1023-1028 (1990).
2. The effect of TNF antibodies on synovial cell IL-1 production has been determined in rheumatoid arthritis patients. Cultures of mononuclear cells extracted from the synovium or synovial fluid of rheumatoid joints produce cytokines for up to 6 days without extrinsic stimulation. In patients with rheumatoid arthritis, synovial cell IL-1 production in culture was significantly reduced by anti-TNF antibody. These results suggest that TNF plays a pivotal role in the induction of IL-1 in rheumatoid joints. See. F.M. Brennan et al., Lancet, vol. 2 (8657), pp. 244-247 (1989).
3. TNF is present at detectable levels in rheumatoid synovial fluids. See. F.S. Di Giovine et al., Ann. Rheum. Dis., vol. 47, pp. 768-776 (1988).

Idiopathic inflammatory bowel disease (IBD) includes two syndromes: ulcerative colitis and Crohn's disease. Crohn's disease is characterized by a granulomatous inflammatory reaction involving the full-thickness of the wall of the terminal ileum or colon, whereas ulcerative colitis is a nonspecific inflammatory response limited to the mucosa and submucosa of the colon. Although there are differences in the pathology of the two syndromes and the etiology of both remains obscure, there is a belief that the two diseases represent variable tissue or immunologic responses to a common etiologic agent. A variety of immunologic disorders have been identified in patients with IBD. One hypothesis is that the immune system of patients is reacting abnormally or inappropriately to antigens, such as bacterial products, to which everyone is commonly exposed. Synthesis of high levels of TNF in the gut may contribute to inflammatory cellular infiltration in IBD. The TNF-mediation of IBD is supported by the following:
1) In two studies with rats, necrosis of the gastrointestinal tract was induced by intravenous bolus administration of TNF The effects of TNF seemed to be dose related. Doses exceeding 0.6 mg/kg induced gross and microscopic necrosis of the small bowel. The bowel exhibited segmental ischemia with regions of frank hemorrhage or necrosis. The cecum appeared particularly sensitive to TNF Histological sections of non-necrotic intestinal tract also showed inflammatory changes with invasions of the submucosa and muscularis mucosa by polymorphonuclear leukocytes. The epithelium was denuded in a focal distribution throughout the bowel. See, X.M. Sun et al., J. Clin. Invest., vol. 81, pp. 1328-1331 (1988); and K.J. Tracey, et al. Science, vol. 234, pp. 470-474 (1986).
2) In the Tracey study, infusion of 4mg of a neutralizing mouse monoclonal antibody directed against human TNF to rats one hour before infusion of TNF not only fully protected the animals from lethal doses of the TNF, but also prevented the development of the lesions typically induced by TNF.
3) Isolates from biopsies of children with Crohn's disease showed elevated frequencies of TNFα secreting cells in all individuals assayed with a spot ELISA technique. Isolates from normal children did not show this increase. In ulcerative colitis, four out of eight children had increased production of TNFα. These results suggest that TNFα is an important mediator of inflammation in the human gut. See, T.T. MacDonald, et al. Clin. Exp. Immunol., (ENGLAND) vol. 81, pp. 301-305 (1990).

Septic shock is a condition associated with massive bacterial invasions. It is commonly believed that shock due to Gram negative infections is brought on, at least in part, by the presence of bacterial endotoxins (lipopolysaccharides). Septic shock is a relatively common cause of mortality in the hospital setting. At present there are few treatment options for patients suffering from septic shock, and the treatments available are generally supportive in nature.

Septic shock is characterized by various symptoms including a drop in mean arterial blood pressure (MAP), a decrease in cardiac output, tachycardia, tachypnea, lacticacidemia and leukopenia. Various cytokines, including TNF, have been implicated in the mediation of septic shock, although the specific etiology of the disease is not fully understood.

There are several lines of evidence which suggest that TNFs may play a role in the mediation of septic shock:
1. The administration of TNF to animals has been shown to induce a shock-like state. See, Everhaerdt et al., Biochem. Biophys. Res. Commun., vol. 163, pg. 378 (1989). It has been shown that the administration of lipopolysaccharides to mice mimics the symptoms of septic shock in the animals. Animals so treated have been found to have increased levels of circulating TNF alpha. See, Parillo et al., Ann. Intern. Med., vol. 9, pp. 28-31 (1988); and Carswell et al., Proc. Natl. Acad. Sci.. USA, vol. 72, pp. 3666-3670 (1975).
2. The administration of antibodies against TNF alpha has been shown to reduce the lethal effects of high doses of endotoxin in mice and monkeys. See, Tracey et al., Nature, vol. 330, pp. 662-664 (1987); Buetler et al., Science, vol. 229, pp. 869-871 (1985).
3. An additional study was conducted wherein the blood serum of children suffering from gram negative septicemia was analyzed for TNF alpha concentration. This study showed that elevated levels of TNF alpha were found in 91 % of the patients examined. In addition, TNF alpha serum levels were significantly higher in patients who died than in the survivors. Firardin et al., New England J. of Med., vol. 319, pp. 397-400 (1988).

The inventors hereof were led to propose that substances which interfere with the activity of TNFs could be effective compounds for the treatment of TNF-mediated diseases, as defined above.

The inventors of the present invention identified a class of compounds, referred to herein as TNF inhibitors, that prevent and treat TNF mediated diseases. In currently pending U.S. Patent application Serial No. 555,274, filed July 19, 1990, a preferred class of naturally occurring proteinaceous TNF inhibitors and a method for manufacturing a substantial quantity of the same with a high degree of purity are described. In particular, the aforementioned application describes in detail two subsets of TNF inhibitors referred to as 30kDa TNF inhibitor and 40kDa TNF inhibitor. In addition to the full-length 40kDa TNF inhibitor protein, two truncated, yet biologically-active forms of the 40kDa TNF inhibitor have also been produced. The full-length 40kDa TNF inhibitor is the TNF inhibitor having a molecular weight of about 40kDa on SDS-PAGE that may be isolated from medium conditioned by human U937 cells or from human urine. Full-length 40kDa TNF inhibitor may be glycosylated as is the naturally-occurring protein; or nonglycoslylated as is the protein recombinantly expressed from a bacterial expression system. The truncated proteins, in which 51 and 53 carboxyl termini amino acids have been removed from the full-length protein, are referred to respectively as 40kDa TNF inhibitor Δ51 and 40kDa TNF inhibitor Δ53.

30kDa TNF inhibitor has been shown to exhibit inhibition activity against TNF alpha. The 40kDa TNF inhibitors, including full-length 40kDa TNF inhibitor, 40kDa TNF inhibitor Δ51 and 40kDa TNF inhibitor Δ53, have been shown to exhibit inhibition activity against both TNF alpha and TNF beta.

Currently pending U.S. Patent Application Serial No. 07/669,862, filed March 15, 1991, describes a number of modified TNF inhibitor species. Muteins of TNF inhibitors are prepared where selected amino acid residue(s) are replaced with cysteine residues. Such muteins may then be site-selectively reacted with functionalized polyethylene glycol (PEG) units to create TNF inhibitor PEG species. In particular, a 30kDa TNF inhibitor mutein referred to as C105 is described, wherein the asparagine at position 105 of 30kDa TNF inhibitor is replaced with cysteine. In one further embodiment, the mutein proteins may be reacted with bifunctionalized PEG units to form bivalent "dumbbell" species wherein two TNF inhibitor muteins are attached via a single PEG chain.

### SUMMARY OF THE INVENTION

The present invention relates to a pharmaceutical composition comprising a TNF Inhibitor modified by the addition of a high molecular weight repeating polymeric moiety material in order to form a physiologically compatible, slow-release formulation.

Furthermore, the present invention relates to the use of a therapeutically effective amount of a TNF inhibitor for preparing a medicament for treating or preventing osteoarthritis, ankylosing spondylosis, gonococcal arthritis, Reiter's disease arthritis and gout.

Preferred TNF inhibitors of the present invention are naturally-occurring proteins and truncated forms of naturally-occurring proteins. The naturally occurring proteins are preferred because they pose a relatively low risk of producing unforseen side effects in patients treated therewith. In some embodiments of this invention, muteins of protein TNF inhibitors where only a small number of amino acid residues differ from the natural protein sequence are also preferred TNF inhibitors.

A preferred class of TNF inhibitors are human TNF binding proteins. The preferred TNF inhibitors appear to be soluble fragments of TNF receptor proteins. Those TNF inhibitors which are preferred in the practice of the present invention are selected from the group consisting of 30kDa TNF inhibitor and, 40kDa TNF inhibitor, and said 40kDa TNF inhibitor is further selected from the group consisting of full-length 40kDa TNF inhibitor, 40kDa TNF inhibitor Δ51 and 40kDa TNF Inhibitor Δ53. Also preferred are proteins which have been modified, for example, by the addition of polyethylene glycol (PEG) or any other repeat polymer to increase their circulating half-life and/or to decrease immunogenicity. TNF inhibitors that act as receptor antagonists to TNF are also included within the scope of this invention.

While the production of TNF inhibitors may be achieved by extraction from naturally available sources, such as by isolation from human urine, a preferred method of TNF inhibitor production is by recombinant DNA technology. Recombinant DNA technology is preferred in part because it is capable of producing comparatively higher amounts of TNF inhibitors at greater purities.

Additional preferred TNF inhibitors include muteins of 30kDa TNF inhibitor wherein selected amino acids of 30kDa TNF inhibitor are replaced with cysteine. Such muteins may be used as TNF inhibitors, or they may be reacted with polyethylene glycol (PEG) to form TNF inhibitor PEG compounds, containing one or two TNF inhibitors per molecule. In the most preferred embodiment, the TNF inhibitor is a bivalent species formed in a reaction between a mutein 30kDa TNF inhibitor and a bifunctionalized PEG precurser. The preferred mutein is C105 30kDa TNF inhibitor, where residue 105 of 30kDa TNF inhibitor is replaced with a cysteine residue.

It is to be understood that both the foregoing general description and the following detailed description are exemplary and explanatory only and are not restrictive of the invention as claimed.

### BRIEF DESCRIPTION OF THE DRAWINGS

FIG. 1 depicts the mortality rates as a function of dose of human recombinant interteukin-1 beta (hrIL-1β) administered with a constant dose of 300 µg/kg of human recombinant Tumor Necrosis Factor alpha (hrTNFα). Groups of six mice were dosed with differing amounts of hrIL-1β. Mortality was assessed at 72 hours after subcutaneous injection of the cytokine mixture. Values given are means ± standard error of the mean (S.E.M.) for three experiments.
FIG. 2 depicts the surface temperature as a function of time post administration of 300 ug/kg of hrTNFα and 2,500 ug/kg of hrIL-1β injected subcutaneously at time zero.
FIG. 3 depicts the surface temperature as a function of time as in Figure 2, wherein -•-•- represents mice given two intraperitoneal injections of 40kDa TNF inhibitor Δ53 at 30 minutes before and 30 minutes after subcutaneous administration of the cytokine mixture. Total 40kDa TNF inhibitor Δ53 administered was 200 mg/kg. *denotes statistically significant differences between TNF inhibitor treated and untreated cytokine-injected mice as assessed by the unpaired t-test (p<0.05).
FIG. 4 depicts the mortality as a function of time in the experiment depicted in FIG. 3.
FIG. 5 depicts the surface temperature as a function of time as in FIG. 2, wherein -- - --represents mice given six intraperitoneal injections of 40kDa TNF inhibitor Δ53 at 30 minutes before, and 30 minutes, 3 hours, 6 hours, 9 hours and 12 hours after administration of the cytokine mixture. Total 40kDa TNF inhibitor A53 administered was 600 mg/kg. * denotes statistically significant differences between TNF inhibitor treated and PBS injected-mice as assessed by the unpaired t-test (p<.001).
FIG. 6 depicts the mortality as a function of time in the experiment depicted in FIG. 5.
FIG. 7 depicts the surface temperature as a function of time as in FIG. 2, wherein -●-●- represents mice given ten intraperitoneal injections of 40kDa TNF inhibitor Δ53 at 30 minutes before, and 30 minutes, 3 hours, 6 hours, 9 hours, 12 hours, 15 hours, 18 hours and 24 hours after subcutaneous administration of the cytokine mixture. Total 40kDa TNF inhibitor Δ53 administered was 1,000 mg/kg.
FIG. 8 depicts the mortality as a function of time of the experiment depicted in FIG. 7.
FIG. 9 depicts the body weights as a function of time in the experiment depicted in FIG. 7.

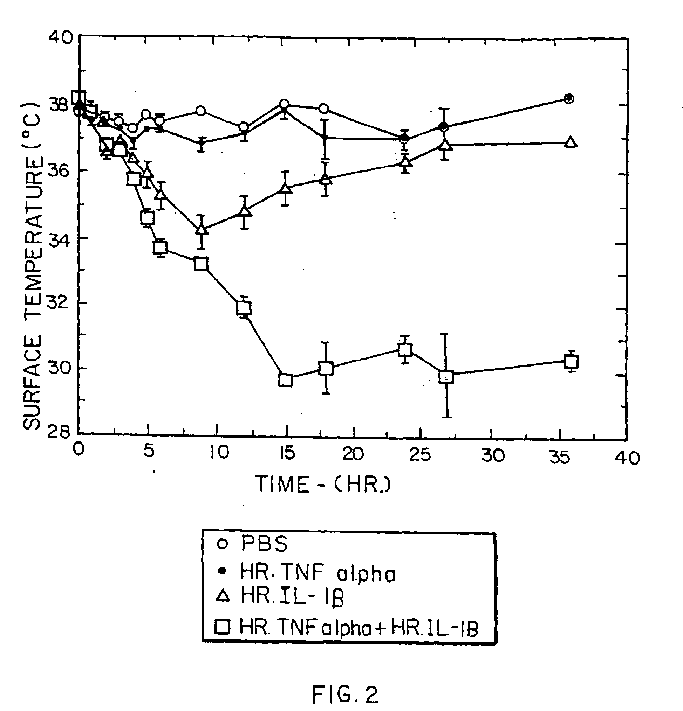

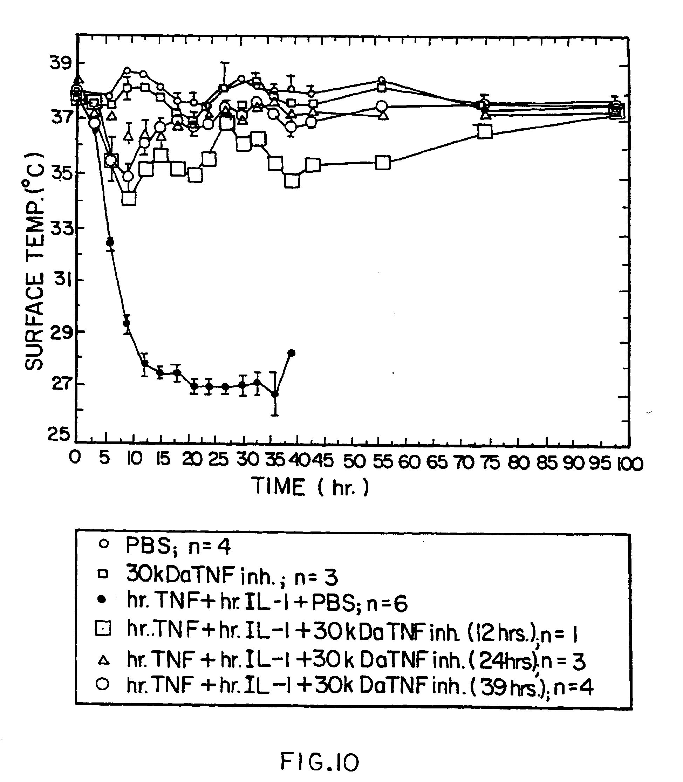

FIG. 10 depicts surface temperature as a function of time as described in FIG. 2, wherein: -□ -□ - represents a mouse given 6 intraperitoneal injections of 30kDa TNF inhibitor at 30 minutes before and 30 minutes, 3 hours, 6 hours, 9 hours and 12 hours after subcutaneous administration of the cytokine mixture (a total of 240 mg/kg); --△-△- represents mice given 10 intraperitoneal injections of 30kDa TNF inhibitor at 30 minutes before and 30 minutes, 3 hours, 6 hours, 9 hours, 12 hours; 15 hours, 18 hours, 21 hours and 24 hours after subcutaneous administration of the cytokine mixture (a total of 400 mg/kg); and --o-o- represent mice given 15 intraperitoneal injections of 30kDa TNF inhibitor at 30 minutes before, and 30 minutes, 3 hours, 6 hours, 9 hours, 12 hours, 15 hours, 18 hours, 21 hours, 24 hours, 27 hours, 30 hours, 33 hours, 36 hours and 39 hours after subcutaneous administration of the cytokine mixture (a total of 600 mg/kg).
FIG. 11 depicts the mortality as a function of time in the experiment depicted in FIG. 10.
FIG. 12 depicts the body weights as a function of time in the experiment depicted in FIG. 10.
FIG. 13 depicts the increase in joint diameter as a function of time post reactivation with Streptococcal Cell Wall (SCW) on day 21, according to Method 1 in Example 3 below.
FIG. 14 depicts the maximum change in joint diameter during the 72 hour interval after reactivation with SCW in the experiment depicted in Fig. 13. *denotes statistically significant differences compared with the PEG 3400 control group by the unpaired t test, t = 4.36, p < 0.001.
FIG. 15 depicts the maximum change in joint diameter during the 72 hour interval after reactivation with SCW according to Method 2 in Example 3 below.
FIG. 16 depicts the percentage inhibition of endotoxin-stimulated neutrophilic influx into bronchoalveolar spaces of 30kDa TNF inhibitor - treated rats as described in Example 4 below.
FIG. 17 depicts the effects of intratracheal instillation of five doses of 30kDa TNF inhibition on the number of neutrophils migrating into the alveolar spaces in response to an endotoxic challenge in the experiment depicted in Fig. 16. * denotes statistically significant differences from the untreated control rats as assessed by the unpaired t test, p < 0.01.

### DETAILED DESCRIPTION OF PREFERRED EMBODIMENTS

Reference will now be made in detail to the presently preferred embodiments of the invention, which, together with the following examples, serve to explain the principles of the invention.

As noted above, the present invention relates to the use of therapeutic agents for the preparation of medicaments for preventing and treating TNF mediated diseases in patients suffering therefrom. While the primary goal of this invention is to provide the use of therapeutic agents for the preparation of medicaments for preventing and treating human diseases, the disclosure provided herein gives instruction of general physiological use, and veterinary uses are therefor also included within the scope of this invention.

A disease or medical indication is to be considered to be a TNF-mediated disease if the spontaneous or experimental disease is associated with elevated levels of TNF in bodily fluids or in tissues adjacent the focus of the disease or indication within the body. TNF mediated diseases may also be recognized by the following two conditions: 1) pathologic findings associated with a disease can be mimicked experimentally in animals by the administration of TNF; and 2) the pathology induced in experimental animal models of the disease can be inhibited or abolished by treatment with agents which inhibit the action of TNF Many TNF-mediated diseases satisfy two of these three conditions and others will satisfy all three conditions. A non-exclusive list of TNF-mediated diseases includes adult respiratory distress syndrome, pulmonary fibrosis, arthritis, inflammatory bowel disease and septic shock

In one embodiment, preferred TNF inhibitors of the present invention are naturally occurring proteins that serve as TNF binding proteins. The naturally-occurring proteins are preferred in part because they pose a comparatively low risk of producing unforeseen and undesirable physiological side effects in patients treated therewith.

For purposes of the specification and claims, a protein is deemed to be "naturally-occurring" if it or a substantially equivalent protein can be found to exist normally in healthy humans. "Naturally-occurring" proteins specifically includes forms of proteins found to exist in healthy humans that are partially truncated at the carboxyl terminus of such proteins, as well as nonglycoslylated forms of proteins that exist in glycosylated forms in healthy humans. "Naturally-occurring" proteins may be obtained by recombinant DNA methods as well as by isolation from cells which ordinarily produce them. "Naturally-occurring" also encompasses proteins that contain an N-terminal methionyl group as a consequence of expression in E. Coli.

"Substantially equivalent" as used throughout the specification and claims is defined to mean possessing a very high degree of amino acid residue homology (See generally M. Dayhoff, Atlas of Protein Sequence and Structure, vol. 5, p. 124 (1972), National Biochemical Research Foundation, Washington, D.C., specifically incorporated herein by reference) as well as possessing comparable biological activity.

Among the preferred TNF inhibitors of the present invention are the naturally-occurring proteins that exist in vivo as binding proteins of TNF that have previously been described in a currently pending United States patent application. This application is U.S. Patent Application Serial No. 07/555,274 filed July 19, 1990, of Brewer et al., which is entitled "Tumor Necrosis Factor (TNF) Inhibitor and Method for Obtaining the Same.".

There are two distinct forms of preferred TNF inhibitors, each disclosed and described in the aforementioned Brewer et al. application. The first of these is 30kDa TNF inhibitor, which has been identified in and isolated from at least a medium conditioned by human U937 cells and from human urine. 30kDa TNF inhibitor is approximately 30kDa on SDS-PAGE, and elutes from a DEAE CL6B column at about 80 millimolar NaCl in Tris buffer, pH 7.5. The 30kDa TNF inhibitor has been shown to inhibit the activity of TNF alpha and has little effect on the activity of TNF beta. The naturally occurring protein is glycosylated. Nonglycosylated 30kDa TNF inhibitor also exhibits TNF inhibitory activity.

The second form of preferred TNF inhibitors is 40kDa TNF inhibitor, which has also been identified and isolated from at least a medium conditioned by human U937 cells and human urine. 40kDa TNF inhibitor is approximately 40kDa on SDS-PAGE, and elutes from a DEAE column at about 100 millimolar NaCl in Tris buffer, pH 7.5. The 40kDa TNF inhibitor has been shown to inhibit the activity of both TNF alpha and TNF beta. 40kDa TNF inhibitor is also a glycoprotein and, again, the nonglycosylated protein exhibits TNF inhibitory activity.

The nucleic acid sequences of the genes encoding both 30kDa TNF inhibitor and 40kDa TNF inhibitor and the amino acid sequences of both proteins are given in the Brewer et al. application. The present invention encompasses nonglycosylated forms of the TNF inhibitors as well as certain truncated forms of the naturally-occurring proteins as described below. In a further embodiment, the TNF inhibitors are modified by attachment of one or more polyethylene glycol (PEG) or other repeating polymeric moieties.

Three forms of the 40kDa TNF inhibitor have been recombinantly produced by expression in E. Coli. Each of these forms, referred to as full-length 40kDa TNF inhibitor, 40kDa TNF inhibitor A51 and 40kDa TNF inhibitor Δ53 (along with the glycosylated full-length 40kDa TNF inhibitor as isolated from medium conditioned by human U937 cells and human urine, in glycosylated and nonglycosylated forms, all of which are collectively referred to herein as 40kDa TNF inhibitor) are described in the Brewer et al. application. The A51 protein is a truncated version of the native protein wherein 51 amino acid residues at the carboxyl terminus of the mature protein are removed. The Δ53 protein is a truncated version of the mature protein wherein 53 amino acid residues at the carboxyl terminus of the native protein are removed. Naturally-occurring 40kDa TNF inhibitor (glycosylated), and the nonglycosylated inhibitors (native, Δ51 and A53) have substantially the same TNF inhibitory activity.

Methods for producing the Brewer et al. inhibitors are also disclosed in the above-mentioned application. One disclosed method consists of isolating the inhibitors from various sources, such as human urine and medium conditioned by human U937 cells. A second disclosed method involves isolating the genes responsible for coding the inhibitors, cloning the gene in suitable vectors and cell types, and expressing the gene to produce the inhibitors. The latter method, which is exemplary of recombinant DNA methods in general, is a preferred method of the present invention. Recombinant DNA methods are preferred in part because they are capable of achieving comparatively higher amounts at greater purities.

Also included within the scope of this invention are TNF inhibitor muteins and muteins that have been modified as described in U.S. patent application Serial No. 07/669,862, filed March 15. 1991. One preferred mutein is 30kDa TNF inhibitor wherein position 105 is a cysteine. A preferred pegylated TNF inhibitor is a "dumbbell" species wherein two C105 30kDa TNF inhibitors are attached to a polyethylene glycol (PEG) moiety. In a most preferred embodiment, the PEG chain has a molecular weight of 20,000. The preparation of the dumbbell compound is described in Example 5 below.

Preferably, the above described TNF inhibitors are produced by the aforementioned method in "substantially pure" form. By "substantially pure" it is meant that the inhibitor, in an unmodified form, has a comparatively high specific activity. It is to be recognized, however, that derivatives of TNF inhibitors may have different specific activities. In a preferred embodiment of the present invention, a therapeutic composition comprising at least one of 30kDa TNF inhibitor or 40kDa TNF inhibitor is administered in an effective amount to patients suffering from TNF mediated diseases.

Additional TNF inhibitors include compounds capable of competing with TNF for TNF receptor sites. Such compounds include receptor antagonists. Other TNF inhibitors include compounds and proteins which block in vivo synthesis or extracellular release of TNF. Such compounds include agents which affect transcription or translation of TNF genes or processing of TNF preproteins.

Because it is possible that the inhibitory function of the preferred inhibitors is imparted by one or more discrete and separable portions, it is also envisioned that the TNF inhibitory fragments of the TNF-inhibitors may be used to prepare a pharmaceutical preparation.

The therapeutic composition of the present invention is preferably administered parenterally by injection, although other effective administration forms, such as intraarticular injection, inhalant mists, orally active formulations, transdermal iontophoresis or suppositories, are also envisioned. One preferred carrier is physiological saline solution, but it is contemplated that other pharmaceutically acceptable carriers may also be used. In one preferred embodiment it is envisioned that the carrier and the TNF inhibitor constitute a physiologically-compatible, slow-release formulation. The primary solvent in such a carrier may be either aqueous or non-aqueous in nature. In addition, the carrier may contain other pharmacologically-acceptable excipients for modifying or maintaining the pH, osmolarity, viscosity, clarity, color, sterility, stability, rate of dissolution, or odor of the formulation. Similarly, the carrier may contain still other pharmacologically-acceptable excipients for modifying or maintaining the stability, rate of dissolution, release, or absorption of the TNF inhibitor. Such excipients are those substances usually and customarily employed to formulate dosages for parenteral administration in either unit dose or multi-dose form.

Once the therapeutic composition has been formulated, it may be stored in sterile vials as a solution, suspension, gel, emulsion; solid, or dehydrated or lyophilized powder. Such formulations may be stored either in a ready to use form or requiring reconstitution immediately prior to administration: The preferred storage of such formulations is at temperatures at least as low as 4°C and preferably at -70°C. It is also preferred that such formulations containing TNF inhibitor are stored and administered at or near physiological pH. It is presently believed that administration in a formulation at a high pH (i.e. greater than 8) or at a low pH (i.e. less than 5) is undesirable.

Preferably, the manner of administering the formulations containing TNF inhibitor for systemic delivery is via subcutaneous, intramuscular, intravenous, intranasal or vaginal or rectal suppository. Preferably the manner of administration of the formulations containing TNF inhibitor for local delivery is via intraarticular, intratracheal, or instillation or inhalations to the respiratory tract. In addition it may be desirable to administer the TNF inhibitor to specified portions of the alimentary canal either by oral administration of TNF inhibitor in an appropriate formulation or device or by suppository or enema.

In certain embodiments, the administration is designed in order to create a preselected concentration range of TNF inhibitor in the patient's blood stream. It is believed that the maintenance of circulating concentrations of TNF inhibitor of less than 0.01 ng per ml of plasma may not be an effective composition white the prolonged maintenance of circulating levels in excess of 10 µg per ml may have undesirable side effects.

A preferred dosage range for the treatment of TNF mediated diseases and more particularly for the treatment of TNF mediated septic shock is between about 0.1-200 mg per kg of patient body weight per 24 hours administered in equal doses between about 4-15 times per 24 hours. In a more preferred embodiment, the dosage is between about 0.1-100 mg per kg of patient body weight per 24 hours administered in equal doses every 3 hours. In the most preferred embodiment 1-50 mg per kg of patient body weight per 24 hours is equally administered every 3 hours. In a preferred embodiment, administration will continue 12 to 60 hours. In a most preferred embodiment administration will continue for at least 24 hours. The frequency of dosing and the optimal dose will depend on pharmacokinetic parameters of the TNF inhibitor in the formulation used.

In an additional preferred mode for the treatment of TNF mediated diseases and more particularly for the treatment of TNF mediated septic shock, an initial intravenous bolus injection of TNF is administered followed by a continuous intravenous infusion of TNF inhibitor until circulating TNF levels are no longer elevated. Serum TNF alpha levels may be ascertained by commercially available immunoassay test kits. The initiation of treatment for TNF mediated septic shock should be begun, under either mode of treatment, as soon as possible after septicemia or the chance of septicemia is diagnosed. For example, treatment may be begun immediately following surgery or an accident or any other event that may carry the risk of initiating septic shock.

Preferred modes for the treatment of TNF mediated diseases and more particularly for the treatment of TNF mediated arthritis include: 1) a single intraarticular injection of TNF inhibitor given periodically as needed to prevent or remedy flare up of arthritis; and 2) periodic subcutaneous injections of TNF inhibitor.

Preferred modes for the treatment of TNF mediated diseases and more particularly for the treatment of TNF mediated adult respiratory distress syndrome include: single or multiple intratracheal administrations of TNF inhibitor; and 2) bolus or continuous intravenous infusion of TNF inhibitor.

it is also contemplated that certain formulations containing TNF inhibitor are to be administered orally. Preferably, TNF inhibitor which is administered in this fashion is encapsulated. The encapsulated TNF inhibitor may be formulated with or without those carriers customarily used in the compounding of solid dosage forms. Preferably, the capsule is designed so that the active portion of the formulation is released at that point in the gastrointestinal tract when bioavailability is maximized and pre-systemic degradation is minimized. Additional excipients may be included to facilitate absorption of the TNF inhibitor. Diluents, flavorings, low melting point waxes, vegetable oils, lubricants, suspending agents, tablet disintegrating agents, and binders may also be employed.

Regardless of the manner of administration, the specific dose is calculated according to the approximate body weight of the patient. Further refinement of the calculations necessary to determine the appropriate dosage for treatment involving each of the above mentioned formulations is routinely made by those of ordinary skill in the art and is within the ambit of tasks routinely performed by them without undue experimentation, especially in light of the dosage information and assays disclosed herein. These dosages may be ascertained through use of the established assays for determining dosages utilized in conjunction with appropriate dose-response data.

It should be noted that the TNF inhibitor formulations described herein may be used for veterinary as well as human applications and that the term "patient" should not be construed in a limiting manner. In the case of veterinary applications, the dosage ranges should be the same as specified above.

It is understood that the application of teachings of the present invention to a specific problem or environment will be within the capabilities of one having ordinary skill in the art in light of the teachings contained herein. Examples of representative uses of the present invention appear in the following examples.

The following examples describe the application of the present invention to one of the TNF mediated diseases described herein. The differences, if any, between the treatment of patients suffering from other TNF mediated diseases from the treatment of patients suffering from TNF-mediated septic shock would be readily and routinely identified by one of ordinary skill in the art. The ability to alleviate the effects of TNF mediated septic shock by administering a TNF inhibitor as shown in the following examples, shows that the administration of a TNF inhibitor will be equally effective in treating all diseases that are mediated by TNF, as defined herein.

### Example 1 : Demonstration of the Protective Effects of Human Recombinant 40kDa TNF inhibitor Δ53 in a Murine model of Cytokine Induced Septic Shock

### A. Protocol for the Induction of Systemic Shock in Mice

A modification of a murine model of septic shock developed by Everaerdt, et al. has been utilized in this Example to demonstrate the therapeutic efficacy of TNF inhibitor against septic shock. Everaerdt et al. Biochem. Biophys. Res. Commun., vol 163, pg. 378 (1989). In this model the synergistic action of a combination of cytokines, human recombinant Tumor Necrosis Factor α (hrTNFα) and human recombinant Interleukin-1β (hrIL-1β) produces septic shock in mice characterized by profound hypothermia and then death.

Lethal septic shock was induced in C57BI/6 female mice, 8-12 weeks old, at 18-21 grams body weight, by the administration of hrTNFα and hrIL-1β. Human recombinant Tumor Necrosis Factor was prepared generally according to the procedure described in Shirai et al., Nature (1985) vol. 313, pp. 803-806,; also see the Brewer, et al. patent application. Human recombinant Interleukin-1β was prepared generally according to the procedure described in Kronheim et al., Biotechnology (1986) vol. 4, pp. 1078-1082. The cytokines were tested for LPS according to the procedures described in United States Pharmocopia XXII, National Formulary XVII, January 1, 1990, pp. 1493-1495. Injections of the cytokines were given as a single bolus, subcutaneously, on the dorsal surface of the mice, in 100µl volumes. The 40kDa TNF inhibitor Δ53 was prepared according to the recombinant DNA method described in the Brewer et al. application and was given in multiple intraperitoneal doses, in volumes of 300µl or less per injection.

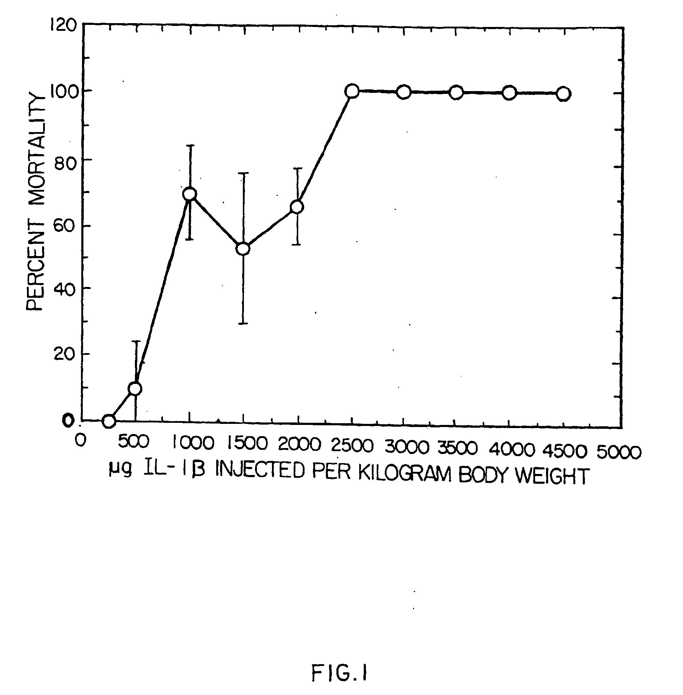

Preliminary experiments were performed to determine the ratio of hrIL-1β to hrTNFα required to induce a mortality of 100% (figure 1). Doses are expressed as µg/kg body weight. While holding the hrTNFα dosage constant at 300µg/kg, increasing amounts of hrIL-1β were added to the injection bolus. Results, given as µg hrIL-1β/kg:% mortality, were as follows; 250:0, 500:10, 1500:50, 2000:67, 2500:100. All doses greater than 2500µg/kg produced 100% lethality. In all subsequent experiments the cytokines were used at 2500µg/kg hrIL-1β and 300µg/kg hrTNFα, and delivered as a single subcutaneous bolus on the dorsal surface.

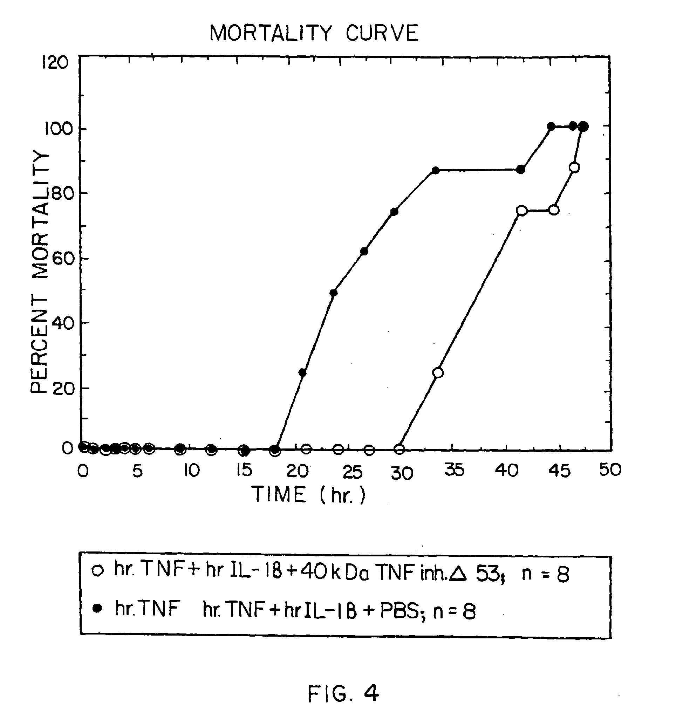

The combined administration of hrTNFα and hrIL-1β caused in mice a profound hypothermia (figure 2) and then death at approximately 18 to 30 hours post injection (figure 4). In this Example, a decrease in body temperature was used as a measurable indicator of the severity of the induced shock Body temperature was estimated by use of First Temp infrared hand held scanner (Intelligent Medical Systems, Carlsbad, CA). The degree of hypothermia correlated directly with the severity of other clinical signs (i.e., shaking, lethargy, loss of skin resiliency, hunched posture). The body temperature of the animals dropped from a normal value of 39°C to as low as 28°C at approximately 15 hours post injection.

In contrast, injections of either cytokine alone did not cause a similar degree of hypothermia (figure 2) and were without lethal effect. Single bolus subcutaneous injections of hrIL-1β at 2500µg/kg caused a transitory drop in body temperature to 34°C reaching that nadir at 12 to 16 hours post injection. No mortality was observed. hrTNFα at 300µg/kg did not produce a measurable effect on the body temperature of mice, nor was any mortality observed.

### B. Effects of 40kDa TNF inhibitor Δ53 on cytokine induced shock.

40kDa TNF inhibitor Δ53 was administered to mice injected with the cytokine mixture (hrIL-1β and hrTNFα in the amounts described above). A series of experiments was performed in which groups of six mice were treated with the Inhibitor. Protein (in this experiment, 40kDa TNF inhibitor Δ53) for increasingly longer periods. The amount of Inhibitor Protein per injection was held constant, while the total number of injections increased. Therefore, the absolute amount of the Inhibitor Protein used in the three experiments varied with the duration of the therapeutic regime. Body temperature and mortality were assessed for each experiment.

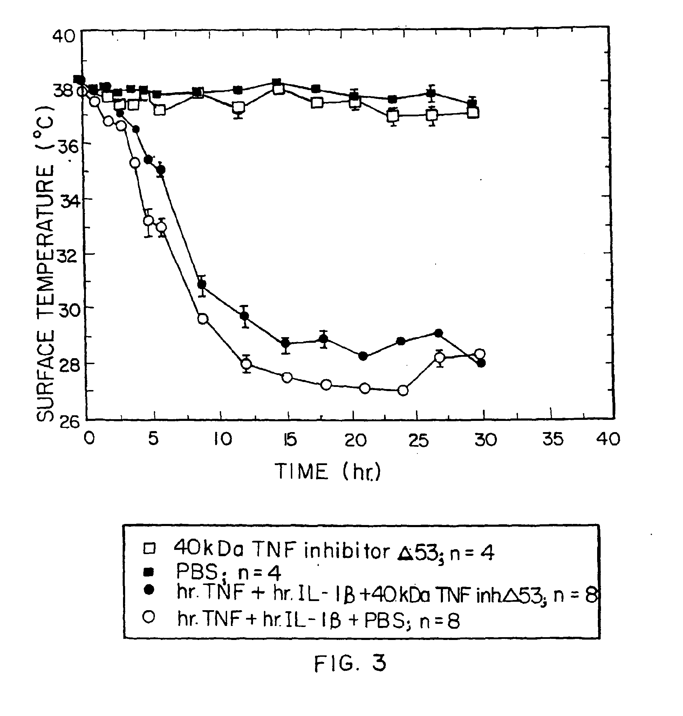

In the first experiment Inhibitor Protein was injected intraperitoneally 30 minutes before and 30 minutes after the cytokine bolus (figure 3). Each injection contained 100 mg/kg of Inhibitor Protein in a 300µl volume for a total of 200 mg/kg. Of the cytokine-shocked mice, those in the group receiving the Inhibitor Protein showed a significant reduction of the hypothermia for a 4 to 6 hour period after the administration of the cytokines as compared to the untreated group (figure 3). Onset of mortality was delayed about 12 hours in the treated group (figure 4). In both groups, however, mortality was 100% at 48 hours post injection.

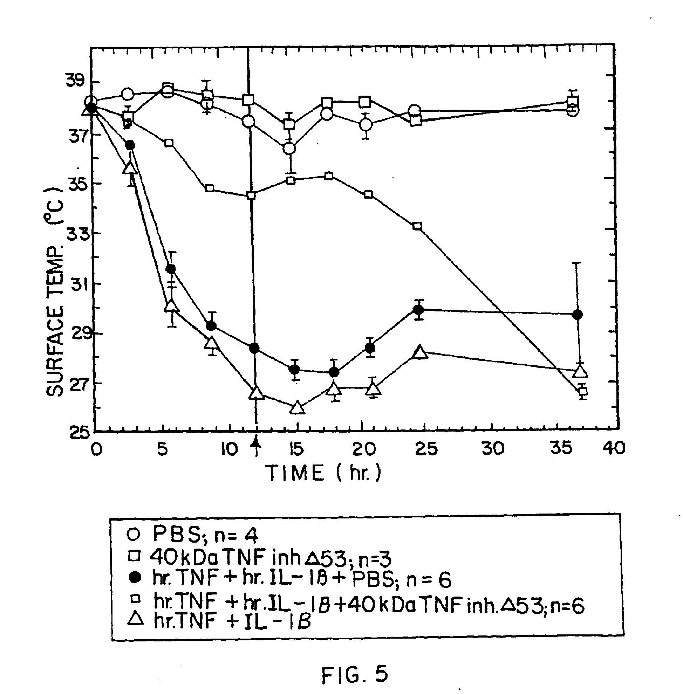

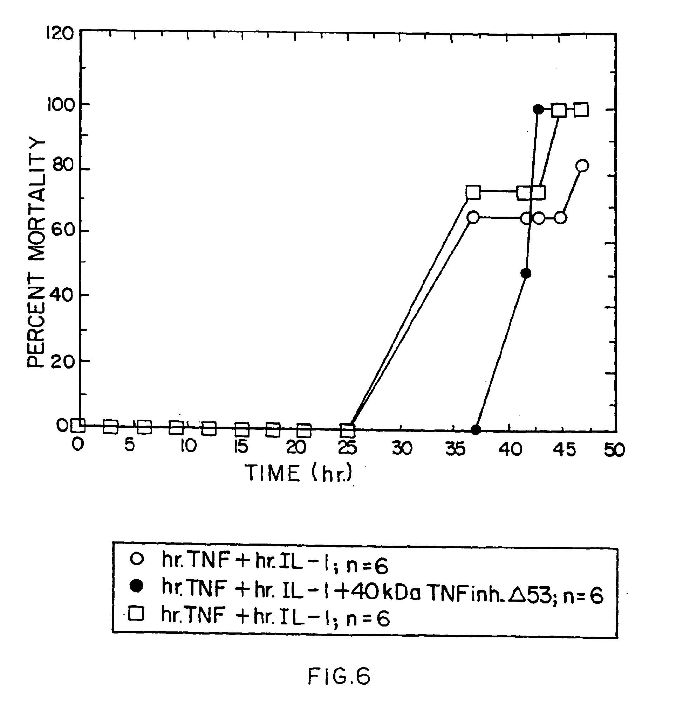

In the second experiment the therapy was continued for a total of 12 hours post injection (figure 5). The injection times relative to the time of cytokine injection were; -30 minutes, +30 minutes, 3 hours, 6 hours, 9 hours, and 12 hours. This regimen resulted in a total dose of Inhibitor Protein of 600 mg/kg. After cytokine injection, the untreated group developed the characteristic hypothermia with a nadir of 26°C at 15 hours. The first deaths were seen at 36 hours (figure 6). Mortality was 100% by 43 hours. In contrast the group treated with Inhibitor Protein was protected against the severe hypothermia for about 24 hours after the injection of the cytokine mixture. Body temperature reached an initial nadir of 35.5°C at 12 hours. At that time, therapy with the Inhibitor Protein had ended. Body temperature rose slightly during the next six hours and then plummeted to 26°C by 36 hours. Relative to the untreated group, the onset of mortality was delayed in the treated group. The first deaths were observed at 42 hours post injection and reached 100% by 44 hours.

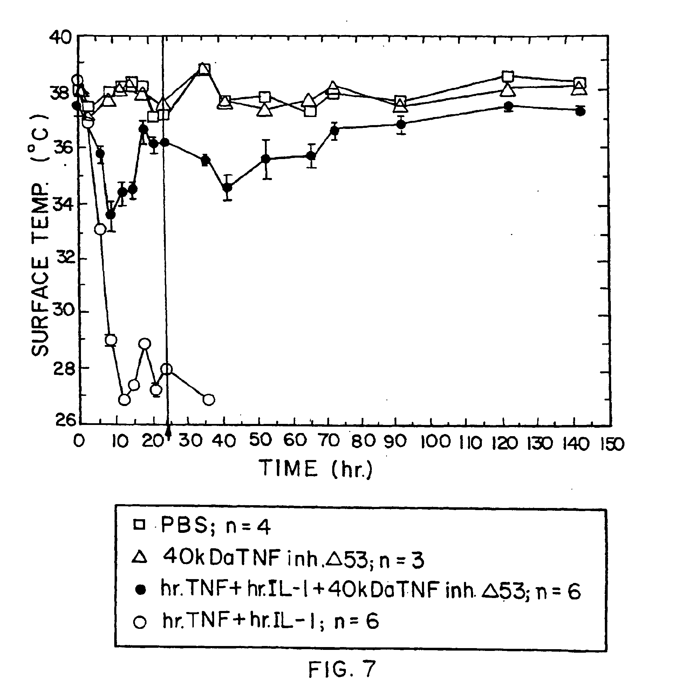

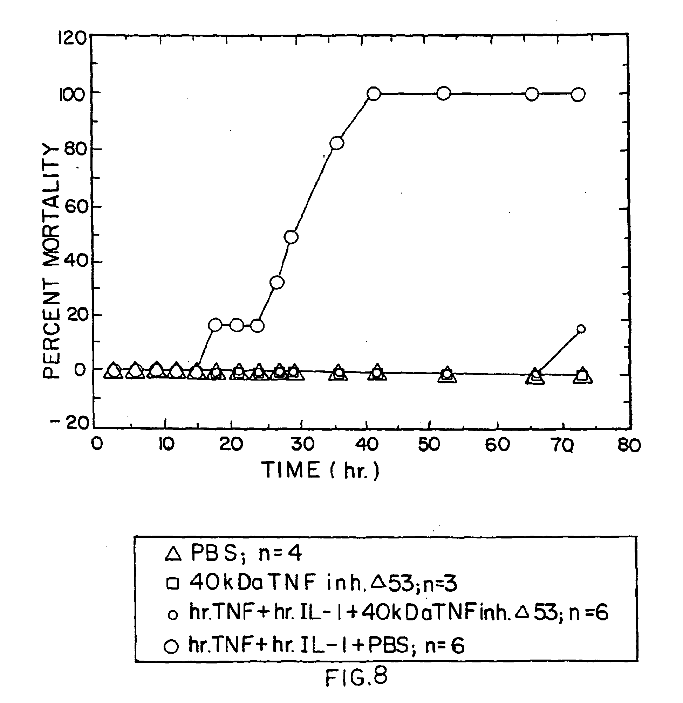

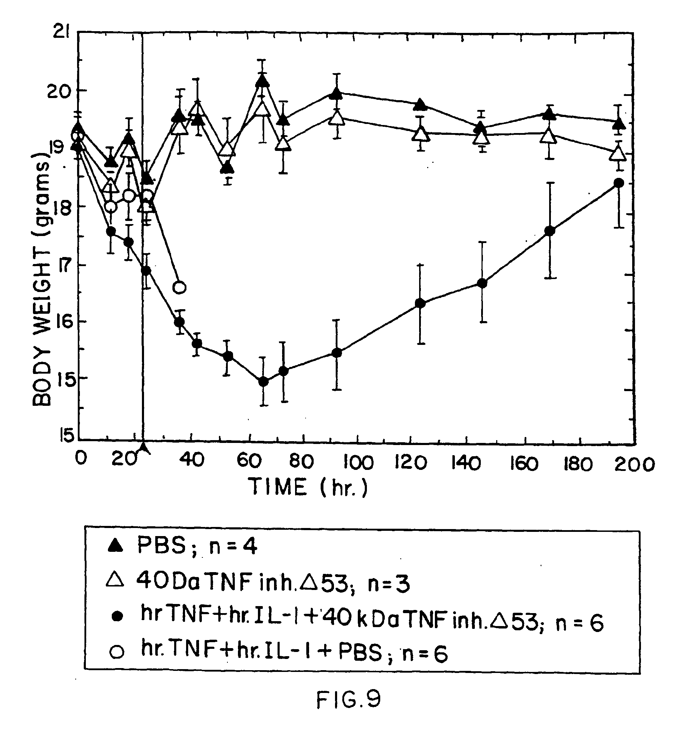

The third experiment extended the above regimen from 12 hours to 24 hours post cytokine injection (figure 7). The additional intraperitoneal injections were administered at 15, 18, 21, and 24 hours after the injection of the cytokine mixture. The resultant total dosage was 1000 mg/kg. The untreated group receiving hrIL-1β and hrTNFα followed the same course as in the second experiment. Body temperature reached a nadir of 27°C at 12 hours. Mortality began at 18 hours post injection and was at 100% by 42 hours (figure 8). In contrast the group receiving Inhibitor Protein was protected against the severe hypothermia and lethality. The average body temperature declined to only 33.5°C by 10 hours post cytokine injection and subsequently rose to near normal by 24 hours at the cessation of Inhibitor Protein treatment (figure 7). By hour 42 (18 hours after cessation of therapy) the body temperature transiently declined to 34.5°C, and remained slightly sub-normal until 90 hours after injection of the cytokines. Mortality in this group was one out of six. The single fatality was unexplained. However, the animal appeared abnormal throughout the experiment, perhaps due to a misplaced intraperitoneal injection. The body weights of these animals were recorded in order to assess full recovery from the cytokine induced shock (figure 9). In the group treated with Inhibitor Protein, the average weight declined to 79% of the original weight by 62 hours. However, by 200 hours post cytokine injection the average weight had returned to that of the control group injected with phosphate buffered saline (PBS). The animals appeared normal in behavior and external appearance. By prolonging therapy with Inhibitor Protein for 24 hours, we were able to reverse the profound hypothermia and lethality seen in cytokine induced model of septic shock in mice. The duration and frequency of the administration of the inhibitor would be compatible with the intensive care given a septic shock patient.

### C. Controls showing non-effects of fluid therapy and the non-toxic nature of the Inhibitor alone.

In the last three experiments described above, Inhibitor Protein (40kDa TNF inhibitor Δ53) was given in volumes of 200 to 300µl per injection. It was possible that the fluid volume of the Inhibitor Protein in itself might affect a therapy. To test that possibility, hypothermia and lethality were compared in one group of mice which received only the cytokines and a second group which received cytokines plus the intraperitoneal injection of PBS in volumes and frequencies identical to the Inhibitor Protein treated group (figure 5). No significant differences in hypothermia or mortality were observed. In subsequent experiments, PBS was administered intraperitoneally to the untreated cytokine shocked mice.

A separate control was included to assess the effects of Inhibitor Protein alone. In all experiments a group of mice were injected with only Inhibitor Protein in identical volumes and frequencies as those given to the treated, cytokine-shocked group. No mortality or alterations of body temperature were observed in the Inhibitor Protein control groups (figures 3,5,7).

### Example 2: Demonstration of the Protective Effects of human recombinant 30kDa TNF inhibitor in a murine model of Cytokine Induced Septic Shock.

### A Protocol for the Induction of Murine Septic Shock

The same murine model of septic shock described in Example 1 was utilized to demonstrate the therapeutic efficacy of 30kDa TNF inhibitor. Lethal shock was induced in C57B1/6 female mice, 8-12 weeks old, weighing 18-21 grams, by the combined subcutaneous administration of hrTNFα at 300 µg/kg and hrIL-1β at 2,500 µg/kg. Mortality was 100% at these doses. The cytokines were delivered as a single subcutaneous bolus on the back of the neck

In this example, a decrease in body temperature was used as a measurable indicator of the severity of the induced shock. The degree of hypothermia correlated directly with the severity of clinical signs (i.e., shaking, lethargy, loss of skin resiliency, hunched posture). After the combined administration of hrTNFα and hrlL-1β, body temperature dropped from a normal value of 39°C to as low as 28°C at approximately 15 hours post injection (figure 2). Temperature was estimated with a First Temp^{®} infrared hand held scanner (Intelligent Medical Systems, Carlsbad, CA). In contrast, injections of either hrTNFα or hrIL-1β alone did not cause a similar degree of hypothermia and were without lethal effects (figure 2). The human recombinant 30kDa TNF inhibitor utilized in this Experiment was prepared according to the methods described in the Brewer et al. patent application.

### B. Effects of 30kDa TNF inhibitor on Cytokine Induced Septic Shock

Inhibitor Protein (in this example 30kDa TNF inhibitor) was given intraperitoneally at 40 mg/kg per injection to a group of 8 mice for periods of either 12, 24, or 39 hours after the subcutaneous administration of hrTNFα and hrIL-1β. The first group, containing one mouse, was treated with Inhibitor Protein at 30 minutes before and at 30 minutes, 3 hours, 6 hours, 9 hours, and 12 hours after the injection of the cytokines. This mouse received a total of 240 mg/kg of the Inhibitor Protein given in six injections. In the next group, treatment was extended another 12 hours with the additional injections given at 3 hour intervals. The three mice in this group each received during the 24 hour period a total of 400 mg/kg of Inhibitor Protein given in ten injections, In the next group, treatment was extended an additional 15 hours with the added injections again being given at 3 hour intervals. The four mice in this group each received during the 39 hour period a total of 600 mg/kg given in 15 injections.

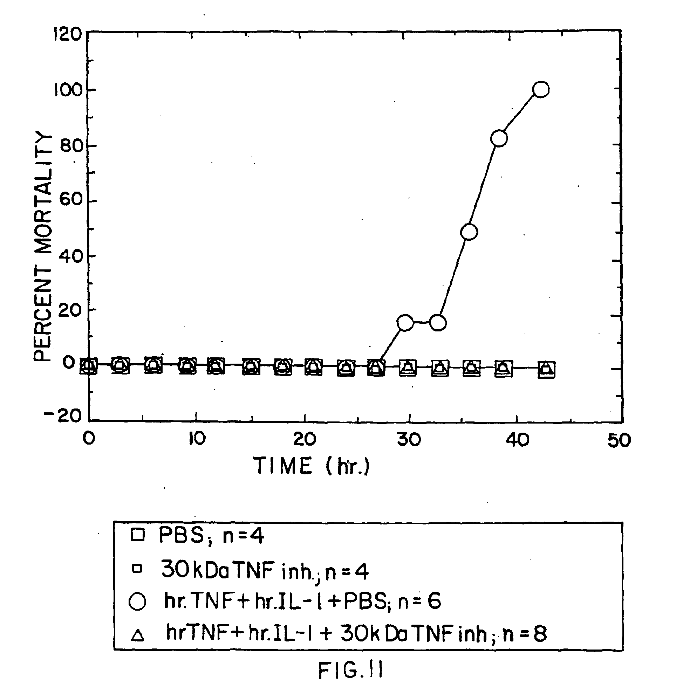

Body temperature (figure 10) and mortality (figure 11) were followed in the treated and untreated mice. The mean temperature of the untreated mice dropped to 27°C at 12 hours and remained at that low level until the mice died at 36 to 42 hours after the administration of the cytokines. In contrast, the body temperature of the mice in all three treatment groups was only moderately reduced and all mice survived the cytokine induced shock. During the first 24 hour period after the administration of the cytokines, body temperature dropped 2 to 4°C by 12 hours in all three groups and then climbed to normal by 24 hours. Thereafter, temperature was maintained at normal levels in the groups receiving either 24 hour or 39 hour Inhibitor Protein treatment, whereas the temperature of the single mouse treated for only 12 hours dropped 3°C at 39°C and subsequently returned to a normal value by 100 hours post cytokine injection.

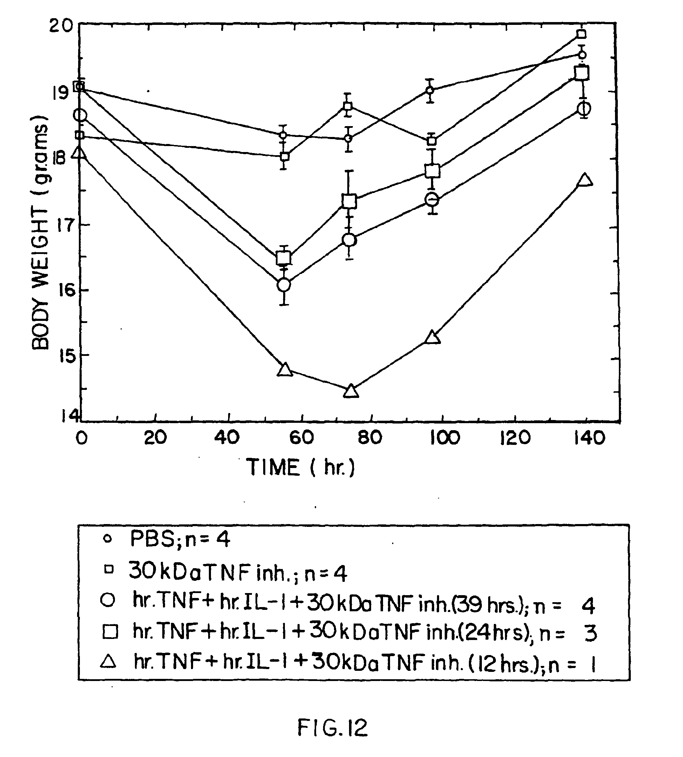

The time required for the body weight to return to time zero values was also used as an index of recovery after cytokine induced shock (figure 12). The body weights of the mice treated with Inhibitor Protein for either 24 or 39 hours dropped to 86% of the time zero values by 60 hours and then returned to normal levels by 140 hours. The body weight of the single mouse treated with the inhibitor for 12 hours reached a low of 81% at a slightly later time, 75 hours, yet attained the time zero value at about 140 hours; the same point as the other two groups.

30kDa TNF inhibitor was effective in protecting mice against a cytokine induced lethal septic shock syndrome when therapy was maintained at 3 hour intervals for periods of either 12, 24, or 39 hours after injection of the cytokine mixture. A similar therapeutic regimen can be readily accomplished in a septic shock patient in an intensive care setting.

### Example 3: Demonstration of the Effects of Human Recombinant 30kDa TNF Inhibitor on Streptococcal Cell Wall (SCW)-Induced Reactivation of SCW-Induced Arthritis in Rats

This experiment employs the model disclosed in Esser, et al., Arthritis and Rheumatism, 28: 1401-1411, (1985). This model is briefly summarized as follows: Streptococcal Cell Wall (SCW) is injected intraarticulary into the ankle joint of Lewis rats. Saline is injected into the contralateral joint to provide a control. After a period of twenty days, in which the initial inflammation dies away, SCW is again administered, this time by intravenous injection. This dose of SCW is insufficient to cause joint inflammation by itself and therefore, has little effect on the saline-injected ankle. In contrast, however, this dose is capable of reactivating inflammation and joint destruction in the ankle previously injected with SCW. To assess the extent of inflammation following the second administration of SCW, the dimensions of the ankle joint are measured daily.

Regarding streptococcal cell wall-induced arthritis, R. L. Wilder in Immunopathogenetic Mechanisms of Arthrits. Chapter 9 entitled "Experimental Animal Models of Chronic Arthritis," comments "the clinical, histological and radiological features of the experimental joint disease closely resemble those observed in adult and juvenile rheumatoid arthritis".

Two methods of treating arthritis were preformed using the rodent model of SCW-induced arthritis.

Method One In one set of experiments, arthritic rats were given a single intraarticular administration of TNF inhibitor in phosphate buffered saline (PBS) using the SCW-induced model of arthritis. Arthritis was induced by injecting each rat on day 0 in the left ankle with SCW (1.8 µg rhamnose equivalence). The right ankle was injected with an equal volume of pyrogen-free saline. Ankle dimensions were measured on days 0, 1, 2, and 7. Swelling of the left ankle on days 1 and 2 (Table 1) reflects the acute phase of SCW-induced arthritis. The right ankle which served as a control for the mechanical trauma associated with the intraarticular injection did not undergo significant swelling (data not shown).

On day 21, after the acute phase of arthritis subsided, the rats were randomly divided into four groups of 9 or 10 rats each, anesthetized, and injected according to the protocol described below. C105 is a mutein of 30kDa TNF inhibitor in which an amino acid substitution was performed for the purpose of creating a site for the covalent attachment of polyethylene glycol (PEG). In C105, asparagine has been replaced with cysteine at position 105 of 30kDa TNF inhibitor. In the C105-PEG 3400 dumbbell (db) molecule, two molecules of C105 are cross-linked by a single PEG molecule with an approximate molecular mass of 3400 daltons. The dumbbell molecule and the pegylation procedures are more fully described in U.S. patent application Serial No. 07/669,862 filed March 15, 1991 and entitled "Site Specific Pegylation of Polypeptides". The procedure for producing C105-PEG 3400 db is given below in Example 5.

| Groups Ankle | Left Ankle | Right |
|---|---|---|
| I- PBS Control | PBS | PBS |
| II- PEG 3400 control | PEG 3400 (0.9 µg/joint) | PEG 3400 (0.9 µg/joint) |
| III- C105-PEG 3400 db | C105-PEG 3400db (10 µg/joint) | PEG 3400 (0.9 µg/joint) |
| IV- C105 | C105 (2 µg/joint) | C105 (2 µg/joint) |

Group I served as the vehicle control for the C105-treated group IV Group II served as the vehicle group for the C105-PEG 3400 db-treated group III. The intraarticular injections were made in a total volume of 10 µl via a 25 gauge needle attached to an automatic pipetter. Immediately after the intraarticular injections of the various treatments, each rat was injected intravenously with SCW (150 µg rhamnose equivalence) in order to reactivate the arthritis. No other treatments were administered to the rats. The diameters of the ankle joints were measured on days 21, 22, 23, and 24.

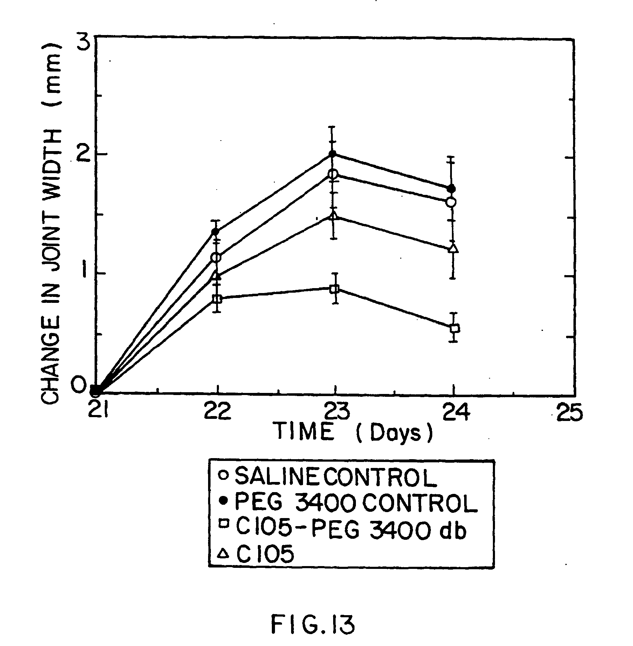

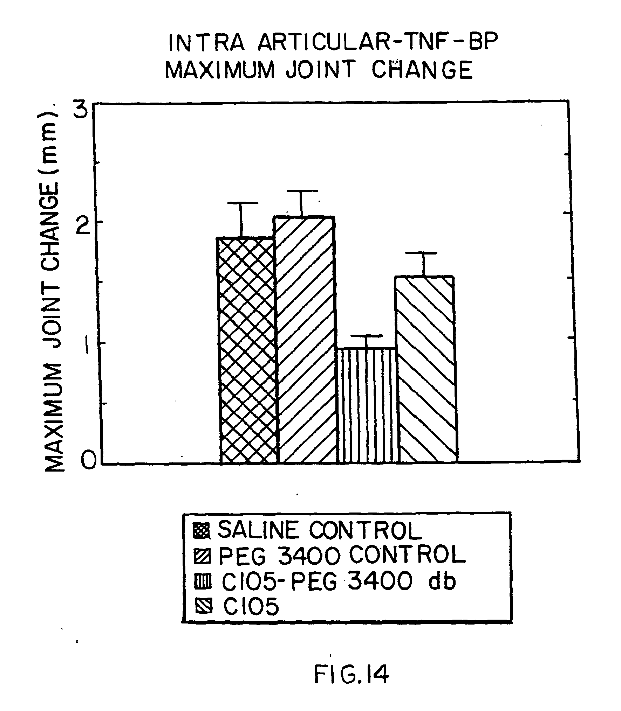

As expected, the left ankles of rats in all groups swelled in response to the intravenous injection of SCW on day 21 (Table 1 and Figure 13). However, the response differed markedly between treatment groups. Whereas the joints of rats in control groups I and II swelled by 30% and 32%, respectively, of their initial dimensions, the corresponding joints in the C105-PEG 3400 db-treated group III increased by only 15% and in the C105-treated group IV by 25%. In Figure 14, the maximum increases in joint diameter during the 72 hour-interval after SCW-induced reactivation are graphed for Groups I, II, III, and IV. A single intraarticular injection of C105-PEG 3400 db caused a statistically significant reduction in swelling of the arthritic joints.

Method Two In a second set of experiments, arthritic rats were given multiple subcutaneous administrations of 30kDa TNF inhibitor in phosphate buffered saline (PBS) using the same SCW model of arthritis. Arthritis was induced by injecting each rat on day 1 in the left ankle with SCW (1.8 µg rhamnose equivalence) and in the right ankle with an equal volume of pyrogen-free saline.

On day 21 after the acute arthritis subsided, the rats were randomly divided into four groups. Each rat was injected intravenously with a second dose of SCW (150 µg rhamnose equivalence) in order to reactivate the arthritis. Within three minutes after the injection of SCW, the nine rats in control group I were treated with PBS and the five rats in each of Groups II, III, and IV were treated with 1, 3, and 9 mg per kg body weight, respectively, of 30kDa TNF inhibitor in PBS. Subcutaneous injections of PBS to Group I or 30kDa TNF inhibitor to Groups II, III, and IV (1, 3, and 9 mg/kg, respectively) were given again at two and six hours post SCW-administration and were repeated every 6 hours during the 42 hour period thereafter. The diameters of ankle joints were measured at 0, 24, 39, 48, 60 and 72 hours after the intravenous injection of SCW.

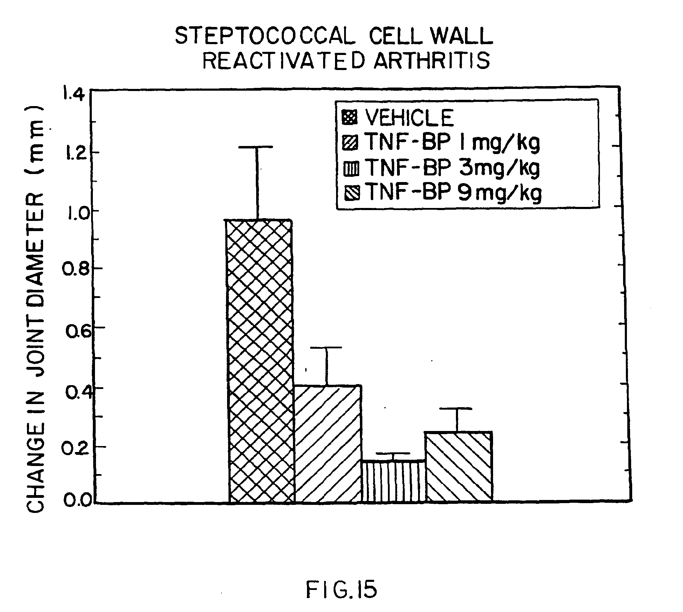

Figure 15 and Table 2 show the maximum changes in joint diameters during the 72 hour period after SCW-induced reactivation of arthritis in the four experimental groups. As expected, the ankles in saline-treated group I swelled in response to the intravenous injection of SCW. However, the maximal swelling in groups II, III, and IV was reduced by 58%, 85%, and 75%, respectively. Table 2 shows the results of statistical analysis of the data from Figure 15 using the t-test on means which have undergone a logarithmic transformation.

### Example 4 : Demonstration of the Effects of Human Recombinant 30kDa TNF Inhibitor on Acute Lung Injury Induced by Endotoxin in Rats as a Method for Treating Adult Respiratory Distress Syndrome (ARDS)

This experiment employs a septic stimulus, endotoxin, to induce acute, neutrophil-mediated pulmonary injury according to the model disclosed in Ulich, et al., American Journal of Pathology 138: 1485-1496, (1991). This model is briefly summarized as follows: Endotoxin is injected intratracheally into the midcervical portion of the trachea of anesthetized rats. After a latent period of six hours, bronchoalveolar lavage (BAL) of the lungs is performed as a terminal procedure. Total and differential white blood cell counts are performed on the BAL fluid. Intratracheal injection of pyrogen-free saline yields BAL fluid with a predominance of alveolar macrophages (about 99%) in low numbers. Intratracheal injection of endotoxin causes a large increase in the number of BAL cells and a predominance of neutrophils. The acute neutrophilic influx into the alveolar space peaks at 6 to 12 hours and is accompanied by the accumulation of protein-containing edematous fluid into the alveolar spaces. TNF is believed to be one of the mediators of endotoxin-induced alveolitis. TNF is present in the BAL fluid after stimulation with endotoxin. The alveolar macrophage is believed to be the source of its synthesis. Moreover, intratracheal injection of exogenous TNF induces an acute intraalveolar neutrophilic exudate which is qualitatively similar to that induced by endotoxin.

Although animal models are not considered to faithfully replicate the human disease, in particular the transition from the acute to chronic phases of ARDS, many animal models are used which approximate the altered pulmonary edema, sequestration of leukocytes, and hypoxemia during the acute phase of lung parenchymal injury during ARDS. J. F. Murray et al., Am. Rev. of Respiratory Disease vol. 138, pp. 720-723 (1991). The acute forms of ARDS occur in the presence of certain identifiable risk factors such as sepsis, aspiration, or multiple blood transfusions. Current investigations emphasize the central role of neutrophilic mediated injury in the pathophysiology of ARDS (Tate, R.M. Am. Rev. Resp. Dis. 128:552-559, (1983)). Toxic products released by the activated neutrophil are believed to damage the alveolar capillary membrane. The permeability of the damaged membrane is greatly increased stimulating the movement of plasma proteins and inflammatory cells into the alveolar spaces. In an experimental ovine model of ARDS of septic origin, neutrophil depletion protected against the development of pulmonary injury (Heflin, A.C. J. Clin. Invest. 68:1253-1260, (1981))".

A method for treating neutrophilic alveolitis in rats by the intratracheal administration of 30kDa TNF inhibitor in PBS was performed. Lung injury was induced by the administration of endotoxin (5 µg per rat) in a total volume of 0.5 ml of sterile PBS through a 27 gauge 1/2 inch needle inserted between tracheal rings in the surgically exposed midcervical region of the trachea. The inoculum was administered slowly into the trachea while monitoring the rate and depth of respiration of the rat. Six hours later, the rats were anesthetized with isoflurane so that a laparotomy could be performed in order to facilitate the lavage of the lungs. The caudal vena cava was severed to decrease the blood content of the lungs. The diaphragm was opened to allow the lungs to expand during lavage. BAL was performed by injecting 40 ml of Hank's balanced salt solution into the bronchoalveolar spaces via an angiocath catheter which was inserted and secured at the site of a midcervical tracheal incision. The inflammatory cellular influx was recovered from the pellet obtained by centrifuging the BAL fluid at 1500 rpm for 15 min. The total number of leukocytes were counted on a Coulter counter. The percentage of polymorphonuclear neutrophils was determined by performing a differential cell count manually on a slide of stained cells.

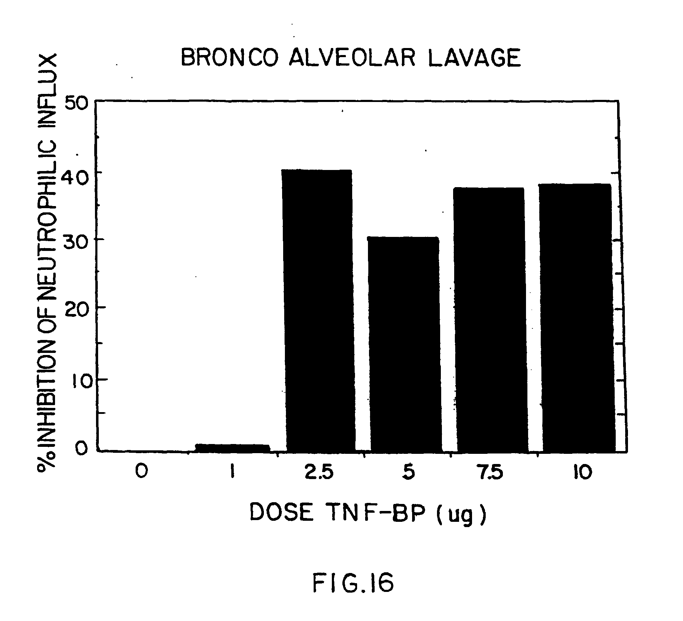

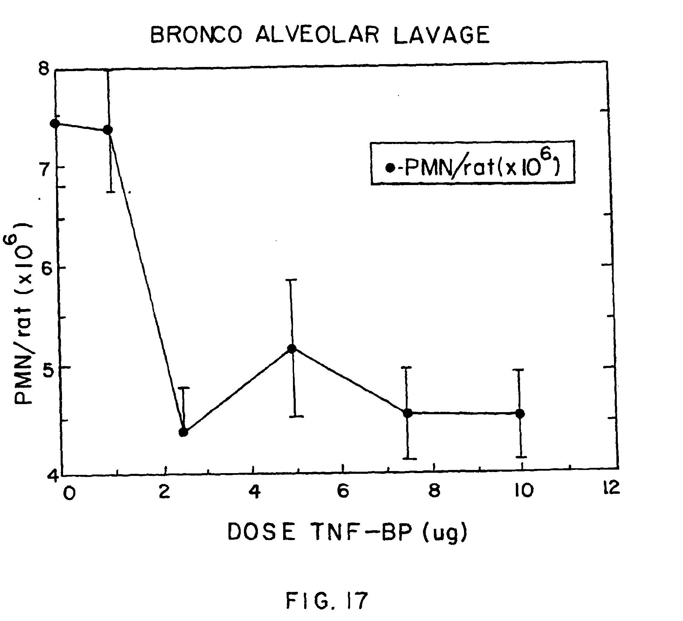

The effects of 30kDa TNF inhibitor on the endotoxin-stimulated influx of cells into the bronchoalveolar spaces were determined by administering 30kDa TNF inhibitor simultaneously with the intratracheal instillation of endotoxin. 30kDa TNF inhibitor was tested in doses ranging between 1 to 10 µg per rat. 30kDa TNF inhibitor at a dose of 1 µg/rat did not reduce the influx of neutrophils into the alveolar spaces. However, the intratracheal administration of 30kDa TNF inhibitor at doses between 2.5 µg to 10 µg per rat caused a maximal reduction in the influx of neutrophils into the alveolar spaces. Compared with the neutrophilic influx in untreated rats, the percentage inhibition of the cellular influx in these 30kDa TNF inhibitor treated-rats was approximately 35% (Figure 16). The total number of neutrophils present in the bronchoalveolar spaces was significantly reduced in rats treated with 2.5 to 10 µg 30kDa TNF inhibitor per rat (Figure 17 and Table 3).

### Example 5: Preparation of C105-PEG 3400 db.

C105-PEG db compounds were prepared by the reaction of the cysteine containing mutein of 30kDa TNF inhibitor C105 with a PEG-bis-maleimide. To prepare the PEG-bis maleimide, PEG-tresylate was prepared from PEG-bis-diol as described by Nilson and Mosbach, in Methods in Enzymology, vol. 104, pp. 56-69, Academic Press, Inc., New York, NY (1984). The amount of sulfonated intermediate was determined by elemental analysis for fluorine. This intermediate was converted to the phthalimide derivative which was subsequently reduced with hydrazine hydrate to the PEG-bis-amine intermediate. These two reactions were carried out by the procedure described by Pillai, et al., J.
Org. Chem., vol. 45 pp. 5364-5370 (1980). The amount of each intermediate was estimated by elemental analysis for nitrogen. In addition, the amount of PEG-bis-amine was determined by reaction with 2,4,6-trinitrobenzene sulfonic acid. The PEG-bis-amine derivatives were converted to the corresponding bis-maleimide products by reaction with maleic anhydride via an adaption of the procedure of Butler and Hartley, in Methods in Enzymology, vol. XXV, pp. 191-199, Academic Press, Inc., New York, NY (1972), and by cyclization of the intermediate using the method described by Wunsch, et al., Biol. Chem. Hoope-Seyler, vol. 366, pp. 56-61 (1985).

The mutein C105 30kDa TNF inhibitor is prepared as described in U.S. patent application serial no. 07/669,862 filed March 15, 1991. C105 30kDa TNF inhibitor at 2-3 mg/ml is treated with a 4-fold molar excess of dithiothreitol (DTT) for 2 hours at ambient temperature. The mutein is then dialyzed against degassed 50 mM HEPES pH 7.0 for 3 hours at 4°C. To create the dumbbell compound - which can be considered a polyethylene glycol (PEG) linked dimer -- the dialyzed mutein is reacted with the PEG-bis-maleimide species. The ratio of mutein to PEG-bis-maleimide varies depending on the molecular mass of the PEG compound. For PEG-bis-maleimide with a molecular weight of about 1900, a 1 to 1 molar ratio was utilized, while for PEG compounds with a molecular weight of 3,400 or 20,000 a 2 to 1 molar ratio of mutein to PEG compound was used. The reactions are incubated for 3-12 hours at ambient temperature.

Following incubation, the PEG-linked dimers were purified from unpegylated mutein and PEG-mutein monomers using MONO-S FPLC in 50mM HoAc pH 4.0 using a 260 mM, 310mM and 350 mM NaCl step gradient. The dumbbell compounds are eluted at the 310 mM NaCl step. Remaining unPEGylated mutein was removed by chromatography on superdex 75. C105-PEG 3400 db refers to a dumbbell compound (a TNF inhibitor PEG-linked dimer) where the TNF inhibitor is the C105 mutein of 30kDa TNF inhibitor, and the PEG unit has a molecular mass of about 3400 daltons.

Although the present invention has been described in connection with preferred embodiments, it is understood that those skilled in the art are capable of making modifications and variations without departing from the scope or spirit of the present invention. Therefore, the foregoing description of preferred embodiments is not to be taken in a limiting sense, and the present invention is best defined by the following claims and their equivalents.

**Table 1**

| Ankle Joint Diameter of Rats injected with SCW and Treated by the Intraarticular injection of Either Saline, Saline + PEG 3400, C105-PEG 3400db, or C105 | | | | | | | | |
|---|---|---|---|---|---|---|---|---|
| Day | Group I (Saline) | | Group II (Saline + PEG 3400) | | Group III (C105-PEG 3400db) | | Group IV (C105) | |
| | Mean | SE | Mean | SE | Mean | SE | Mean | SE |
| 0 | 5.89 ± 0.04 | | 5.97 ± 0.05 | | 5.90 ± 0.06 | | 5.91 ± 0.06 | |
| 1 | 6.66 ± 0.15 | | 6.93 ± 0.13 | | 6.89 ± 0.09 | | 6.67 ± 0.09 | |
| 7 | 6.29 ± 0.09 | | 6.53 ± 0.23 | | 6.29 ± 0.08 | | 6.28 ± 0.03 | |
| | | | | | | | | |
| 21 | 6.20 ± 0.06 | | 6.30 ± 0.06 | | 6.15 ± 0.05 | | 6.14 ± 0.06 | |
| 22 | 7.35 ± 0.18 | | 7.64 ± 0.10 | | 6.94 ± 0.12 | | 7.13 ± 0.16 | |
| 23 | 8.05 ± 0.30 | | 8.30 ± 0.20 | | 7.04 ± 0.13 | | 7.65 ± 0.20 | |
| 24 | 7.83 ± 0.33 | | 8.02 ±0.20 | | 6.71 ± 0.11 | | 7.36 ± 0.25 | |

**Table 2. Maximum changes in joint diameter during the 72 hour period after reactivation of SCW-induced arthritis**

| Group **#** | Change in Joint Diameter | n ° |
|---|---|---|
| | (mm) | |
| I (Saline) | 0.96 ± 0.25 | 9 |
| II (TNF/bp 1 mg/kg) | 0.40 ± 0.13 | 5 |
| III (TNF/bp 3 mg/kg) | 0.14 ± 0.03* | 5 |
| IV (TNF/bp 9 mg/kg) | 0.24 ± 0.08* | 5 |
| Values are expressed as mean ± standard error. | | |
| * statistically significant difference compared to control group I as assessed by the unpaired t test performed on means which have undergone a log transformation) | | |
| Group I vs Group II | t = 1.43 | p = 0.177 |
| Group I vs Group III | t = 3.20 | p = 0.008 |
| Group I vs Group IV | t = 2.19 | p = 0.049 |
| Group I vs Groups III & IV | t = 3.19 | p = 0.005 |
| Group I vs Groups II, III & IV | t = 2.88 | p = 0.009 |

**Table 3**

| Total number of neutrophils recovered from bronchoalveolar fluid of endotoxin-challenged rats | | | |
|---|---|---|---|
| Dose of TNF/bp | # Neutrophils ( x 10⁶) | n | p value |
| 0 | 7.4 ± 0.6 | 8 | |
| 1 | 7.3 ± 0.6 | 4 | > 0.5 |
| 2.5 | 4.3 ± 0.4* | 4 | < 0.01 |
| 5 | 5.7 ± 0.8 | 7 | > 0.2 |
| 7.5 | 4.6 ± 0.4* | 4 | < 0.025 |
| 10 | 4.6 ± 0.4* | 6 | < 0.005 |

| | | | |
|---|---|---|---|
| * Significantly different from the untreated control group as assessed by the unpaired t test. | | | |

## Claims (Claims for the following Contracting State(s): AT, BE, CH, DE, DK, FR, GB, IT, LI, LU, MC, NL, SE)

1. A pharmaceutical composition comprising a TNF inhibitor modified by the addition of a high molecular weight repeating polymeric moiety material in order to form a physiologically compatible, slow release formulation.

2. Use of a therapeutically effective amount of a TNF inhibitor comprising an amino acid sequence selected from the group consisting of
(i) the amino acid sequence of the 30 kDa inhibitor or a TNF inhibitory fragment thereof or a mutein thereof;
(ii) the amino acid sequence of the 40 kDa inhibitor or a TNF inhibitory fragment or mutein thereof;
(iii) the amino acid sequence of the 40 kDa inhibitor Δ53 or a TNF inhibitory fragment or mutein thereof; and
(iv) the amino acid sequence of the 40 kDa inhibitor Δ51 or a TNF inhibitory fragment or mutein thereof
for preparing a medicament for treating or preventing osteoarthritis, ankylosing spondylosis, gonococcal arthritis, Reiter's disease arthritis and gout.

3. Use according to claim 2 wherein said TNF inhibitor has a non-naturally occurring cysteine residue.

4. Use according to claim 3 wherein the non-naturally occurring cysteine residue is at position 105 of the 30 kDa inhibitor

5. Use according to any one of claims 2 to 4, wherein the TNF inhibitor is glycosylated or wherein the TNF inhibitor is not glycosylated.

6. Use according to any one of claims 2 to 4, wherein the TNF-inhibitor has a methionine residue at the N-terminus.

7. Use according to any one of claims 2 to 6, wherein said TNF inhibitor is produced by recombinant DNA methods.

8. Use according to any one of claims 2 to 7, wherein the TNF-inhibitor is substantially pure.

9. Use according to any one of claims 2 to 8, wherein said polypeptide is linked to a high molecular weight repeating polymeric moiety material.

10. Use according to claim 9, wherein the high molecular weight polymeric material is polyethylene glycol.

11. Use according to any one of claims 2 to 10, wherein the medicament comprises a pharmacologically compatible, slow-release formulation.

12. Use according to any one of claims 2 to 11, wherein the medicament allows for intra-articular, subcutaneous, intramuscular, intravenous, intranasal, oral or topical administration of the TNF inhibitor.

13. Use according to any one of claims 2 to 11, wherein the medicament allows for continuous infusion.

## Claims (Claims for the following Contracting State(s): GR)

1. A process for preparing a pharmaceutical composition comprising modifying a TNF inhibitor by the addition of a high molecular weight repeating polymeric moiety material in order to form a physiologically compatible, slow-release formulation.

2. Use of a therapeutically effective amount of a TNF inhibitor comprising an amino acid sequence selected from the group consisting of:
(i) the amino acid sequence of the 30 kDa inhibitor or a TNF inhibitory fragment thereof or a mutein thereof;
(ii) the amino acid sequence of the 40 kDa inhibitor or a TNF inhibitory fragment or mutein thereof;
(iii) the amino acid sequence of the 40 kDa inhibitor Δ53 or a TNF inhibitory fragment or mutein thereof;
(iv) the amino acid sequence of the 40 kDa inhibitor Δ51 or a TNF inhibitory fragment or mutein thereof
for preparing a medicament for treating or preventing osteoarthritis, ankylosing spondylosis, gonococcal arthritis, Reiter's disease arthritis and gout.

3. Use according to claim 2 wherein said TNF inhibitor has a non-naturally occurring cysteine residue.

4. Use according to claim 3 wherein the non-naturally occurring cysteine residue is at position 105 of the 30 kDa inhibitor.

5. Use according to any one of claims 2 to 4, wherein the TNF inhibitor is glycosylated or wherein the TNF inhibitor is not glycosylated.

6. Use according to any one of claims 2 to 4, wherein the TNF-inhibitor has a methionine residue at the N-terminus.

7. Use according to any one of claims 2 to 6, wherein said TNF inhibitor is produced by recombinant DNA methods.

8. Use according to any one of claims 2 to 7, wherein the TNF-inhbitor is substantially pure.

9. Use according to any one of claims 2 to 8, wherein said polypeptide is linked to a high molecular weight repeating polymeric moiety material.

10. Use according to claim 9, wherein the high molecular weight polymeric material is polyethylene glycol.

11. Use according to any one of claims 2 to 10, wherein the medicament comprises a pharmacologically compatible, slow-release formulation.

12. Use according to any one of claims 2 to 11, wherein the medicament allows for intra-articular, subcutaneous, intramuscular, intravenous, intranasal, oral or topical administration of the TNF inhibitor.

13. Use according to any one of claims 2 to 11, wherein the medicament allows for continuous infusion.

## Claims (Claims for the following Contracting State(s): ES)

1. A process for preparing a pharmaceutical composition comprising modifying a TNF inhibitor by the addition of a high molecular weight repeating polymeric moiety material in order to form a physiologically compatible, slow-release formulation.

2. A process for preparing a pharmaceutical composition for treating or preventing osteoarthritis, ankylosing spondylosis, gonococcal arthritis, Reiter's disease arthritis, and gout, comprising using a TNF inhibitor comprising an amino acid sequence selected from the group consisting of
(i) the amino acid sequence of the 30 kDa inhibitor or a TNF inhibitory fragment or a mutein thereof;
(ii) the amino acid sequence of the 40kDa inhibitor or a TNF inhibitory fragment or mutein thereof;
(iii) the amino acid sequence of the 40 kDa inhibitor Δ53 or a TNF inhibitory fragment or mutein thereof; and
(iv) the amino acid sequence of the 40 kDa inhibitor Δ51 or a TNF inhibitory fragment or mutein thereof.

3. The process according to claim 2, wherein said TNF inhibitor has a non-naturally occurring cysteine residue.

4. The process according to claim 3, wherein the non-naturally occurring cysteine residue is at position 105 of the 30 kDa inhibitor.

5. The process according to any one of claims 2 to 4, wherein the TNF inhibitor is glycosylated or wherein the TNF inhibitor is not glycosylated.

6. The process according to any one of claims 2 to 4, wherein the TNF-inhibitor has a methionine residue at the N-terminus.

7. The process according to any one of claims 2 to 6, wherein said TNF inhibitor is produced by recombinant DNA methods.

8. The process according to any one of claims 2 to 7, wherein the TNF-inhibitor is substantially pure.

9. The process according to any of claims 2-8 wherein said polypeptide is linked to a high molecular weight repeating polymeric moiety material.

10. The process according claim 9, wherein the high molecular weight polymeric material is polyethylene glycol.

11. The process according to any one of claims 2 to 10, wherein the medicament comprises a pharmacologically compatible, slow-release formulation.

12. The process according to any one of claims 2 to 11, wherein the medicament allows for intra-articular, subcutaneous, intramuscular, intravenous, intranasal, oral or topical administration of the TNF inhibitor.

13. The process according to any of claims 2 to 12, wherein the medicament allows for continuous infusion.

## Patentansprüche (Patentansprüche für folgende(n) Vertragsstaat(en): AT, BE, CH, DE, DK, FR, GB, IT, LI, LU, MC, NL, SE)

1. Eine pharmazeutische Zusammensetzung, umfassend einen TNF-Inhibitor, der durch die Addition eines aus einer sich wiederholenden Komponente gebildeten Polymer-Materials mit hohem Molekulargewicht modifiziert ist, um eine physiologisch verträgliche Formulierung zur verzögerten Freigabe zu bilden.

2. Verwendung einer therapeutisch wirksamen Menge eines TNF-Inhibitors, der eine Aminosäuresequenz umfasst, die aus folgender Gruppe ausgewählt wird:
(i) die Aminosäuresequenz des 30 kDa-Inhibitors oder eines TNF-inhibitorischen Fragments davon oder eines Muteins davon;
(ii) die Aminosäuresequenz des 40 kDa-Inhibitors oder eines TNF-inhibitorischen Fragments oder Muteins davon;
(iii) die Aminosäuresequenz des 40 kDa-Inhibitors Δ53 oder eines TNF-inhibitorischen Fragments oder Muteins davon; und
(iv) die Aminosäuresequenz des 40 kDa-Inhibitors Δ51 oder eines TNF-inhibitorischen Fragments oder Muteins davon
zur Herstellung eines Arzneimittels zur Behandlung oder Vorbeugung von Osteoarthritis, Spondylitis ankylosans, Arthritis gonorrhoica, mit der Reiter-Krankheit assoziierter Arthritis und Gicht.

3. Verwendung nach Anspruch 2, bei der der TNF-Inhibitor einen natürlich nicht vorkommenden Cystein-Rest aufweist.

4. Verwendung nach Anspruch 3, bei der der natürlich nicht vorkommende Cystein-Rest an der Position 105 des 30 kDa-Inhibitors sich befindet.

5. Verwendung nach einem der Ansprüche 2 bis 4, bei der der TNF-Inhibitor glycosyliert ist oder bei der der TNF-Inhibitor nicht glycosyliert ist.

6. Verwendung nach einem der Ansprüche 2 bis 4, bei der der TNF-Inhibitor am N-Terminus einen Methionin-Rest aufweist.

7. Verwendung nach einem der Ansprüche 2 bis 6, bei der der TNF-Inhibitor durch DNA-Rekombinationsverfahren hergestellt wird.

8. Verwendung nach einem der Ansprüche 2 bis 7, bei der der TNF-Inhibitor im Wesentlichen rein ist.

9. Verwendung nach einem der Ansprüche 2 bis 8, bei der das Polypeptid an ein aus einer sich wiederholenden Komponente gebildetes Polymer-Material mit hohem Molekulargewicht gekoppelt ist.

10. Verwendung nach Anspruch 9, bei der das Polymer-Material mit hohem Molekulargewicht Polyethylenglycol ist.

11. Verwendung nach einem der Ansprüche 2 bis 10, bei der das Arzneimittel eine pharmakologisch verträgliche Formulierung zur verzögerten Freigabe umfasst.

12. Verwendung nach einem der Ansprüche 2 bis 11, bei der das Arzneimittel eine intraartikuläre, subkutane, intramuskuläre, intravenöse, intranasale, orale oder lokale Verabreichung des TNF-Inhibitors erlaubt.

13. Verwendung nach einem der Ansprüche 2 bis 11, bei der das Arzneimittel eine kontinuierliche Infusion erlaubt.

## Patentansprüche (Patentansprüche für folgende(n) Vertragsstaat(en): GR)

1. Ein Verfahren zur Herstellung einer pharmazeutischen Zusammensetzung, umfassend das Modifizieren eines TNF-Inhibitors durch die Addition eines aus einer sich wiederholenden Komponente gebildeten Polymer-Materials mit hohem Molekulargewicht, um eine physiologisch verträgliche Formulierung zur verzögerten Freigabe zu bilden.

2. Verwendung einer therapeutisch wirksamen Menge eines TNF-Inhibitors, der eine Aminosäuresequenz umfasst, die aus folgender Gruppe ausgewählt wird:
(i) die Aminosäuresequenz des 30 kDa-Inhibitors oder eines TNF-inhibitorischen Fragments davon oder eines Muteins davon;
(ii) die Aminosäuresequenz des 40 kDa-Inhibitors oder eines TNF-inhibitorischen Fragments oder Muteins davon;
(iii) die Aminosäuresequenz des 40 kDa-Inhibitors Δ53 oder eines TNF-inhibitorischen Fragments oder Muteins davon; und
(iv) die Aminosäuresequenz des 40 kDa-Inhibitors Δ51 oder eines TNF-inhibitorischen Fragments oder Muteins davon
zur Herstellung eines Arzneimittels zur Behandlung oder Vorbeugung von Osteoarthritis, Spondylitis ankylosans, Arthritis gonorrhoica, mit der Reiter-Krankheit assoziierter Arthritis und Gicht.

3. Verwendung nach Anspruch 2, bei der der TNF-Inhibitor einen natürlich nicht vorkommenden Cystein-Rest aufweist.

4. Verwendung nach Anspruch 3, bei der der natürlich nicht vorkommende Cystein-Rest an der Position 105 des 30 kDa-Inhibitors sich befindet.

5. Verwendung nach einem der Ansprüche 2 bis 4, bei der der TNF-Inhibitor glycosyliert ist oder bei der der TNF-Inhibitor nicht glycosyliert ist.

6. Verwendung nach einem der Ansprüche 2 bis 4, bei der der TNF-Inhibitor am N-Terminus einen Methionin-Rest aufweist.

7. Verwendung nach einem der Ansprüche 2 bis 6, bei der der TNF-Inhibitor durch DNA-Rekombinationsverfahren hergestellt wird.

8. Verwendung nach einem der Ansprüche 2 bis 7, bei der der TNF-Inhibitor im Wesentlichen rein ist.

9. Verwendung nach einem der Ansprüche 2 bis 8, bei der das Polypeptid an ein aus einer sich wiederholenden Komponente gebildetes Polymer-Material mit hohem Molekulargewicht gekoppelt ist.

10. Verwendung nach Anspruch 9, bei der das Polymer-Material mit hohem Molekulargewicht Polyethylenglycol ist.

11. Verwendung nach einem der Ansprüche 2 bis 10, bei der das Arzneimittel eine pharmakologisch verträgliche Formulierung zur verzögerten Freigabe umfasst.

12. Verwendung nach einem der Ansprüche 2 bis 11, bei der das Arzneimittel eine intraartikuläre, subkutane, intramuskuläre, intravenöse, intranasale, orale oder lokale Verabreichung des TNF-Inhibitors erlaubt.

13. Verwendung nach einem der Ansprüche 2 bis 11, bei der das Arzneimittel eine kontinuierliche Infusion erlaubt.

## Patentansprüche (Patentansprüche für folgende(n) Vertragsstaat(en): ES)

1. Ein Verfahren zur Herstellung einer pharmazeutischen Zusammensetzung, umfassend das Modifizieren eines TNF-Inhibitors durch die Addition eines aus einer sich wiederholenden Komponente gebildeten Polymer-Materials mit hohem Molekulargewicht, um eine physiologisch verträgliche Formulierung zur verzögerten Freigabe zu bilden.

2. Ein Verfahren zur Herstellung einer pharmazeutischen Zusammensetzung zur Behandlung oder Vorbeugung von Osteoarthritis, Spondylitis ankylosans, Arthritis gonorrhoica, mit der Reiter-Krankheit assoziierter Arthritis und Gicht, umfassend die Verwendung eines TNF-Inhibitors, der eine Aminosäuresequenz umfasst, die aus folgender Gruppe ausgewählt wird:
(i) die Aminosäuresequenz des 30 kDa-Inhibitors oder eines TNF-inhibitorischen Fragments davon oder eines Muteins davon;
(ii) die Aminosäuresequenz des 40 kDa-Inhibitors oder eines TNF-inhibitorischen Fragments oder Muteins davon;
(iii) die Aminosäuresequenz des 40 kDa-Inhibitors Δ53 oder eines TNF-inhibitorischen Fragments oder Muteins davon; und
(iv) die Aminosäuresequenz des 40 kDa-Inhibitors Δ51 oder eines TNF-inhibitorischen Fragments oder Muteins davon.

3. Verfahren nach Anspruch 2, bei dem der TNF-Inhibitor einen natürlich nicht vorkommenden Cystein-Rest aufweist.

4. Verfahren nach Anspruch 3, bei dem der natürlich nicht vorkommende Cystein-Rest an der Position 105 des 30 kDa-Inhibitors sich befindet.

5. Verfahren nach einem der Ansprüche 2 bis 4, bei dem der TNF-Inhibitor glycosyliert ist oder bei dem der TNF-Inhibitor nicht glycosyliert ist.

6. Verfahren nach einem der Ansprüche 2 bis 4, bei dem der TNF-Inhibitor am N-Terminus einen Methionin-Rest aufweist.

7. Verfahren nach einem der Ansprüche 2 bis 6, bei dem der TNF-Inhibitor durch DNA-Rekombinationsverfahren hergestellt wird.

8. Verfahren nach einem der Ansprüche 2 bis 7, bei dem der TNF-Inhibitor im Wesentlichen rein ist.

9. Verfahren nach einem der Ansprüche 2 bis 8, bei dem das Polypeptid an ein aus einer sich wiederholenden Komponente gebildetes Polymer-Material mit hohem Molekulargewicht gekoppelt ist.

10. Verfahren nach Anspruch 9, bei dem das Polymer-Material mit hohem Molekulargewicht Polyethylenglycol ist.

11. Verfahren nach einem der Ansprüche 2 bis 10, bei dem das Arzneimittel eine pharmakologisch verträgliche Formulierung zur verzögerten Freigabe umfasst.

12. Verfahren nach einem der Ansprüche 2 bis 11, bei dem das Arzneimittel eine intraartikuläre, subkutane, intramuskuläre, intravenöse, intranasale, orale oder lokale Verabreichung des TNF-Inhibitors erlaubt.

13. Verfahren nach einem der Ansprüche 2 bis 12, bei dem das Arzneimittel eine kontinuierliche Infusion erlaubt.

## Revendications (Revendications pour l'(les) Etat(s) contractant(s) suivant(s): AT, BE, CH, DE, DK, FR, GB, IT, LI, LU, MC, NL, SE)

1. Composition pharmaceutique comprenant un inhibiteur de TNF modifié par l'addition d'une substance à motif polymère répétitif de masse moléculaire élevée, pour donner une formulation physiologiquement acceptable, à libération lente.

2. Utilisation d'une quantité thérapeutiquement efficace d'un inhibiteur de THF comprenant une séquence d'aminoacides choisie dans l'ensemble constitué par
(i) la séquence d'aminoacides de l'inhibiteur de 30 kDa ou d'un fragment inhibiteur de TNF ou d'une mutéine de ceux-ci ;
(ii) la séquence d'aminoacides de l'inhibiteur de 40 kDa ou d'un fragment inhibiteur de TNF ou d'une mutéine de ceux-ci ;
(iii) la séquence d'aminoacides de l'inhibiteur Δ53 de 40 kDa ou d'un fragment inhibiteur de TNF ou d'une mutéine de ceux-ci ; et
(iv) la séquence d'aminoacides de l'inhibiteur Δ51 de 40 kDa ou d'un fragment inhibiteur de TNF ou d'une mutéine de ceux-ci
pour la préparation d'un médicament destiné au traitement ou à la prévention de l'ostéoarthrite, de la spondylite ankylosante, de l'arthrite gonococcique, de l'arthrite de Reiter et de la goutte.

3. Utilisation selon la revendication 2, dans laquelle ledit inhibiteur de TNF comporte un résidu cystéine qui n'est pas présent naturellement.

4. Utilisation selon la revendication 3, dans laquelle ledit résidu cystéine qui n'est pas présent naturellement est à la position 105 de l'inhibiteur de 30 kDa.

5. Utilisation selon l'une quelconque des revendications 2 à 4, dans laquelle l'inhibiteur de TNF est glycosylé ou dans laquelle l'inhibiteur de TNF n'est pas glycosylé.

6. Utilisation selon l'une quelconque des revendications 2 à 4, dans laquelle l'inhibiteur de TNF comporte un résidu méthionine à l'extrémité N-terminale.

7. Utilisation selon l'une quelconque des revendications 2 à 6, dans laquelle ledit inhibiteur de TNF est produit par des méthodes d'ADN recombinant.

8. Utilisation selon l'une quelconque des revendications 2 à 7, dans laquelle l'inhibiteur de TNF est pratiquement pur.

9. Utilisation selon l'une quelconque des revendications 2 à 8, dans laquelle ledit polypeptide est lié à une substance à motif polymère répétitif de masse moléculaire élevée.

10. Utilisation selon la revendication 9, dans laquelle la substance polymère de masse moléculaire élevée est le polyéthylèneglycol.

11. Utilisation selon l'une quelconque des revendications 2 à 10, dans laquelle le médicament comprend une formulation pharmacologiquement acceptable, à libération lente.

12. Utilisation selon l'une quelconque des revendications 2 à 11, dans laquelle le médicament permet l'administration intra-articulaire, sous-cutanée, intramusculaire, intraveineuse, intranasale, orale ou topique de l'inhibiteur de TNF.

13. Utilisation selon l'une quelconque des revendications 2 à 11, dans laquelle le médicament permet une perfusion continue.

## Revendications (Revendications pour l'(les) Etat(s) contractant(s) suivant(s): GR)

1. Procédé pour la préparation d'une composition pharmaceutique, comprenant la modification d'un inhibiteur de TNF par l'addition d'une substance à motif polymère répétitif de masse moléculaire élevée, pour donner une formulation à libération lente, physiologiquement acceptable.

2. Utilisation d'une quantité thérapeutiquement efficace d'un inhibiteur de THF comprenant une séquence d'aminoacides choisie dans l'ensemble constitué par
(i) la séquence d'aminoacides de l'inhibiteur de 30 kDa ou d'un fragment inhibiteur de TNF ou d'une mutéine de ceux-ci ;
(ii) la séquence d'aminoacides de l'inhibiteur de 40 kDa ou d'un fragment inhibiteur de TNF ou d'une mutéine de ceux-ci ;
(iii) la séquence d'aminoacides de l'inhibiteur Δ53 de 40 kDa ou d'un fragment inhibiteur de TNF ou d'une mutéine de ceux-ci ; et
(iv) la séquence d'aminoacides de l'inhibiteur Δ51 de 40 kDa ou d'un fragment inhibiteur de TNF ou d'une mutéine de ceux-ci
pour la préparation d'un médicament destiné au traitement ou à la prévention de l'ostéoarthrite, de la spondylite ankylosante, de l'arthrite gonococcique, de l'arthrite de Reiter et de la goutte.

3. Utilisation selon la revendication 2, dans laquelle ledit inhibiteur de TNF comporte un résidu cystéine qui n'est pas présent naturellement.

4. Utilisation selon la revendication 3, dans laquelle le résidu cystéine qui n'est pas présent naturellement est à la position 105 de l'inhibiteur de 30 kDa.

5. Utilisation selon l'une quelconque des revendications 2 à 4, dans laquelle l'inhibiteur de TNF est glycosylé ou dans laquelle l'inhibiteur de TNF n'est pas glycosylé.

6. Utilisation selon l'une quelconque des revendications 2 à 4, dans laquelle l'inhibiteur de TNF comporte un résidu méthionine à l'extrémité N-terminale.

7. Utilisation selon l'une quelconque des revendications 2 à 6, dans laquelle ledit inhibiteur de TNF est produit par des méthodes d'ADN recombinant.

8. Utilisation selon l'une quelconque des revendications 2 à 7, dans laquelle l'inhibiteur de TNF est pratiquement pur.

9. Utilisation selon l'une quelconque des revendications 2 à 8, dans laquelle ledit polypeptide est lié à une substance à motif polymère répétitif de masse moléculaire élevée.

10. Utilisation selon la revendication 9, dans laquelle la substance polymère de masse moléculaire élevée est le polyéthylèneglycol.

11. Utilisation selon l'une quelconque des revendications 2 à 10, dans laquelle le médicament comprend une formulation pharmacologiquement acceptable, à libération lente.

12. Utilisation selon l'une quelconque des revendications 2 à 11, dans laquelle le médicament permet l'administration intra-articulaire, sous-cutanée, intramusculaire, intraveineuse, intranasale, orale ou topique de l'inhibiteur de TNF.

13. Utilisation selon l'une quelconque des revendications 2 à 11, dans laquelle le médicament permet une perfusion continue.

## Revendications (Revendications pour l'(les) Etat(s) contractant(s) suivant(s): ES)

1. Procédé pour la préparation d'une composition pharmaceutique, comprenant la modification d'un inhibiteur de TNF par l'addition d'une substance à motif polymère répétitif de masse moléculaire élevée, pour donner une formulation à libération lente, physiologiquement acceptable.

2. Procédé pour la préparation d'une composition pharmaceutique destinée au traitement ou à la prévention de l'ostéoarthrite, de la spondylite ankylosante, de l'arthrite gonococcique, de l'arthrite de Reiter et de la goutte, comprenant l'utilisation d'un inhibiteur de TNF comprenant une séquence d'aminoacides choisie dans l'ensemble constitué par
(i) la séquence d'aminoacides de l'inhibiteur de 30 kDa ou d'un fragment inhibiteur de TNF ou d'une mutéine de ceux-ci ;
(ii) la séquence d'aminoacides de l'inhibiteur de 40 kDa ou d'un fragment inhibiteur de TNF ou d'une mutéine de ceux-ci ;
(iii) la séquence d'aminoacides de l'inhibiteur Δ53 de 40 kDa ou d'un fragment inhibiteur de TNF ou d'une mutéine de ceux-ci ; et
(iv) la séquence d'aminoacides de l'inhibiteur Δ51 de 40 kDa ou d'un fragment inhibiteur de TNF ou d'une mutéine de ceux-ci.

3. Procédé selon la revendication 2, dans lequel ledit inhibiteur de TNF comporte un résidu cystéine qui n'est pas présent naturellement.

4. Procédé selon la revendication 3, dans lequel le résidu cystéine qui n'est pas présent naturellement est à la position 105 de l'inhibiteur de 30 kDa.

5. Procédé selon l'une quelconque des revendications 2 à 4, dans lequel l'inhibiteur de TNF est glycosylé ou dans lequel l'inhibiteur de TNF n'est pas glycosylé.

6. Procédé selon l'une quelconque des revendications 2 à 4, dans lequel l'inhibiteur de TNF comporte un résidu méthionine à l'extrémité N-terminale.

7. Procédé selon l'une quelconque des revendications 2 à 6, dans lequel ledit inhibiteur de TNF est produit par des méthodes d'ADN recombinant.

8. Procédé selon l'une quelconque des revendications 2 à 7, dans lequel l'inhibiteur de TNF est pratiquement pur.

9. Procédé selon l'une quelconque des revendications 2 à 8, dans lequel ledit polypeptide est lié à une substance à motif polymère répétitif de masse moléculaire élevée.

10. Procédé selon la revendication 9, dans lequel la substance polymère de masse moléculaire élevée est le polyéthylèneglycol.

11. Procédé selon l'une quelconque des revendications 2 à 10, dans lequel le médicament comprend une formulation pharmacologiquement acceptable, à libération lente.

12. Procédé selon l'une quelconque des revendications 2 à 11, dans lequel le médicament permet l'administration intra-articulaire, sous-cutanée, intramusculaire, intraveineuse, intranasale, orale ou topique de l'inhibiteur de TNF.

13. Procédé selon l'une quelconque des revendications 2 à 12, dans lequel le médicament permet une perfusion continue.
